(19) Europäisches Patentamt
European Patent Office
Office européen des brevets

(11) EP 0 551 653 B1

(12) EUROPÄISCHE PATENTSCHRIFT

(45) Veröffentlichungstag und Bekanntmachung des Hinweises auf die Patenterteilung:
**17.03.1999 Patentblatt 1999/11**

(51) Int Cl.6: **C07D 498/04**
// (C07D498/04, 265:00, 209:00)

(21) Anmeldenummer: **92122079.4**

(22) Anmeldetag: **28.12.1992**

(54) **Enantiomerenreine Cis-2-Oxa-5,8-diazabicyclo (4.3.0)nonane sowie Verfahren zu ihrer Herstellung**

Enantiomeric pure cis-2-oxa-5,8-diazabicyclo (4.3.0)nonanes and process for their preparation

cis-2-oxa-5,8-diazabicyclo (4.3.0)nonanes sous forme d'énantiomères pures et procédé pour leur préparation

(84) Benannte Vertragsstaaten:
**AT BE CH DE DK ES FR GB GR IE IT LI LU MC NL PT SE**

(30) Priorität: **10.01.1992 DE 4200415**

(43) Veröffentlichungstag der Anmeldung:
**21.07.1993 Patentblatt 1993/29**

(73) Patentinhaber: **BAYER AG**
**51368 Leverkusen (DE)**

(72) Erfinder:
- **Schenke, Thomas, Dr.**
  **W-5060 Bergisch Gladbach 2 (DE)**
- **Krebs, Andreas, Dr.**
  **W-5068 Odentahl (DE)**
- **Petersen, Uwe, Dr.**
  **W-5090 Leverkusen (DE)**

(56) Entgegenhaltungen:
**EP-A- 0 350 733**

- **CHEMICAL ABSTRACTS, vol. 116, 1992, Columbus, Ohio, US; abstract no. 21064c, NAKAGAWA, SUSUMU ET AL. 'Preparation of 1,8-naphthyridine- or quinolinecarboxylic acid derivatives as antibacterial agents' Seite 581 ;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 551 653 B1

## Beschreibung

Die Erfindung betrifft enantiomereinreine Cis-2-Oxa-5,8-diazabicyclo[4.3.0]nonane und Verfahren zu ihrer Herstellung.

Aus der EP 350 733 und JA 3188-080 sind bereits trans-2-Oxa-5,8-diazabicyclo[4.3.0]nonane bekannt geworden, die als interessante Zwischenprodukte für antibakteriell hochwirksame Verbindungen dienen. 2-Oxa-5,8-diazabicyclo[4.3.0]nonan besitzt zwei chirale Kohlenstoffatome und kann daher in vier stereoisomeren Formen auftreten. Da in biologisch wirksamen Verbindungen häufig die Eigenschaften eines Enantiomeren im Vergleich zu den anderen Stereoisomeren stark variieren können, ist es wünschenswert, die enantiomerenreinen Wirkstoffe bereitzustellen.

Die vorliegende Erfindung beschreibt daher Verfahren zur Herstellung von enantiomerenreine cis-Verbindungen der Formel (I),

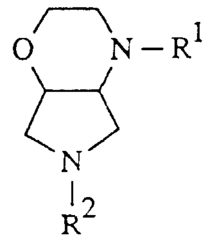

(I)

wobei

$R^1$ für H, $C_1$-$C_3$-Alkyl, Phenyl, Benzyl, 1-Phenylethyl, $C_1$-$C_3$-Alkanoyl, $C_1$-$C_4$-Alkoxycarbonyl, bevorzugt für H, Methyl, Benzyl, 1-Phenylethyl, Acetyl, Ethoxycarbonyl und t-Butoxycarbonyl, besonders bevorzugt für H, Methyl, Benzyl und 1-Phenylethyl steht

und

$R^2$ für H, Benzyl, $C_1$-$C_5$-Alkanoyl, gegebenenfalls ein- oder zweifach durch Halogen oder $C_1$-$C_4$-Alkyl substituiertes Benzoyl, $C_1$-$C_4$-Alkoxycarbonyl, Methansulfonyl, Benzolsulfonyl und Toluolsulfonyl, bevorzugt für H, Benzyl, Acetyl, Benzoyl, Ethoxycarbonyl, t-Butoxycarbonyl, Methansulfonyl und p-Toluolsulfonyl, besonders bevorzugt für H, Benzyl, t-Butoxycarbonyl, Benzoyl und p-Toluolsulfonyl steht.

Es wurde nun gefunden, daß man cis-konfigurierte Verbindungen der Formel (I) dadurch erhält, daß man 2,5-Dihydropyrrolderivate der Formel (II)

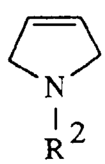

(II)

in welcher $R^2$ die oben angegebene Bedeutung hat, aber nicht Wasserstoff sein kann, durch Umsetzung mit N-Bromsuccinimid oder Brom in Ethylenglykol zu racemischen trans Zwischenstufen der Formel (IIIa),

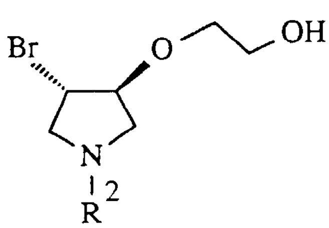

(IIIa)

in welcher $R^2$ die oben angegebene Bedeutung besitzt, aber nicht Wasserstoff sein kann, umsetzt und diese in eine Verbindung der Formel (IIIb)

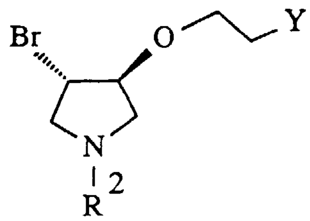

(IIIb)

in welcher $R^2$ die oben angegebene Bedeutung besitzt, aber nicht Wasserstoff sein kann, und Y für Cl, Br oder $O-SO_2-R^3$ steht, wobei $R^3$ Methyl, Phenyl oder Methylphenyl bedeutet, überführt, welche durch Umsetzung mit einem Amin der Formel (IV),

$$R^4-NH_2 \qquad (IV)$$

in welcher

$R^4$ für Wasserstoff, $C_1$-$C_3$-Alkyl, Phenyl, Benzyl oder 1-Phenylethyl steht,

zu einer racemischen cis Verbindung der Formel (V),

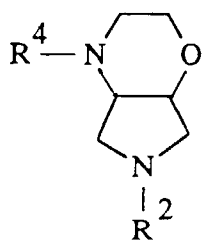

(V)

in welcher
$R^2$ und $R^4$ die oben angegebenen Bedeutungen besitzen, cyclisien wird und anschließend einen gegebenenfalls als Schutzgruppe vorhandenen Rest $R^4$ abspaltet und durch Alkylieren oder Acylieren durch einen Rest $R^1$ ersetzt.

Verbindungen der Formel (IIIb), in welcher Y für Cl oder Br stehen kann, können auch dadurch erhalten werden, daß man eine Verbindung der Formel (II)

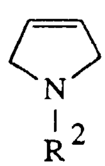

(II)

in welcher $R^2$ die oben angegebene Bedeutung hat, aber nicht Wasserstoff sein kann, mit N-Bromsuccinimid oder Brom in Gegenwart einer Verbindung der Formel (VI)

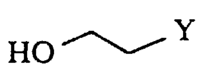

(VI)

in welcher Y für Cl oder Br steht, umsetzt.

Verbindungen der Formel (IIIb), in welcher Y für Br steht, können auch dadurch erhalten werden, daß man eine Verbindung der Formel (II),

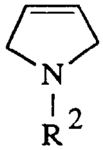

(II)

in welcher $R^2$ die oben angegebene Bedeutung hat, aber nicht Wasserstoff sein kann, mit Brom in Gegenwart von Ethylenoxid umsetzt.

Enantiomerenreine cis Verbindungen der Formel (Ia) oder (Ib)

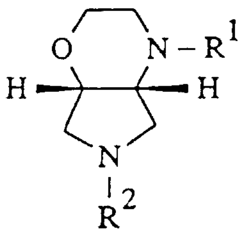

(Ia)

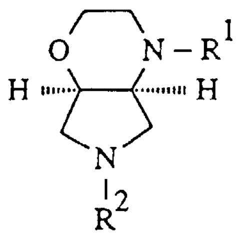

(Ib)

können auch erhalten werden, indem man racemische trans- Verbindungen der Formel (IIIb)

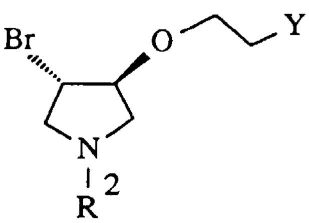

(IIIb)

mit enantiomerenreinem R- oder S-1-Phenylethylamin zu einem Gemisch von jeweils zwei diastereomeren Verbindungen der Formel (VII),

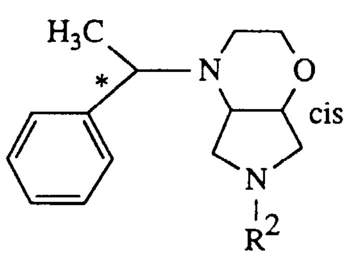

(VII)

in welcher $R^2$ die oben angegebene Bedeutung besitzt, umsetzt und das Gemisch zu enantiomerenreinen Verbindungen auftrennt, den Phenylethylrest gegebenenfalls hydrogenolytisch abspaltet und gegebenenfalls durch einen Rest $R^1$ ersetzt.

Enantiomerenreine cis Verbindungen der Formel (Ia) und (Ib)

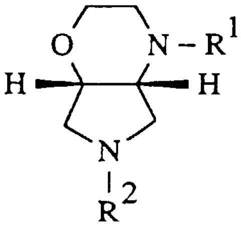

(Ia)

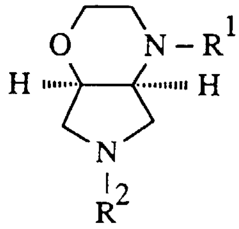

(Ib)

können auch erhalten werden, indem man S,S- oder R,R-Dihydroxypyrrolidinderivate der Formel (VIIIa) oder (VIIIb)

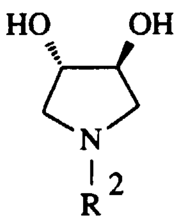

**(VIIIa)**

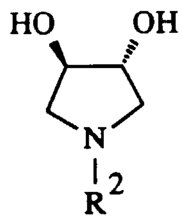

**(VIIIb)**

in denen $R^2$ die oben angegebene Bedeutung hat, aber nicht Wasserstoff sein kann, mit Allylbromid oder Allylchlorid in Gegenwart einer Base zu Verbindungen der Formel (IXa) bzw. (IXb) umsetzt

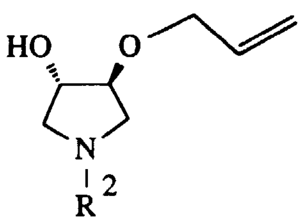

**(IXa)**

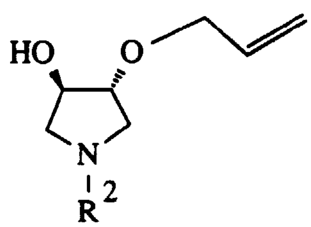

**(IXb)**

die durch Ozonolyse und anschließende Reduktion zu Verbindungen der Formel (Xa) beziehungsweise (Xb) abgebaut werden

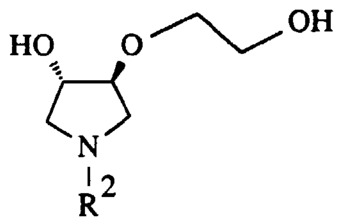

(Xa)

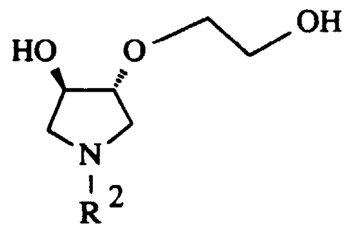

(Xb)

welche durch Acylierung mit Sulfonsäurechloriden $R^3$-$SO_2Cl$ zu Sulfonsäureestern der Formel (XIa) bzw. (XIb) umgesetzt werden, wobei $R^3$ die oben angegebene Bedeutung besitzt,

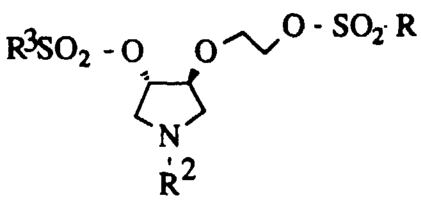

(XIa)

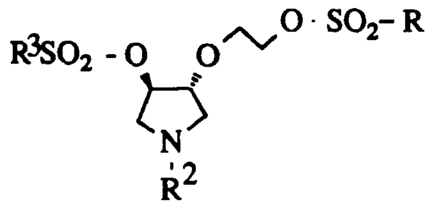

(XIb)

die durch Umsetzung mit Aminen der Formel (IV),

$$R^4\text{-}NH_2 \qquad \text{(IV)}$$

in welcher $R^4$ die oben angegebene Bedeutung besitzt, zu Verbindungen der Formel (XIIa) bzw. (XIIb) cyclisiert werden,

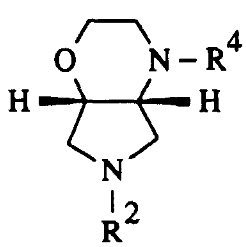

(XIIa)

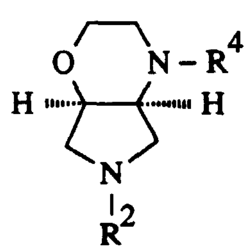

(XIIb)

in welchen man den gegebenenfalls als Schutzgruppe dienenden Rest $R^4$ abspaltet und gegebenenfalls durch Acylieren oder Alkylieren durch einen Rest $R^1$ ersetzt.

Im folgenden werden die einzelnen Schritte des erfindungsgemäßen Verfahrens detaillierter geschildert.

Im ersten Schritt des erfindungsgemäßen Verfahrens werden acylierte oder sulfonylierte 2,5-Dihydropyrrolderivate der Formel (II) zu racemischen trans Zwischenstufen der Formel (III) umgesetzt, wobei $R^2$ die oben angegebene Bedeutung mit Ausnahme von H besitzt.

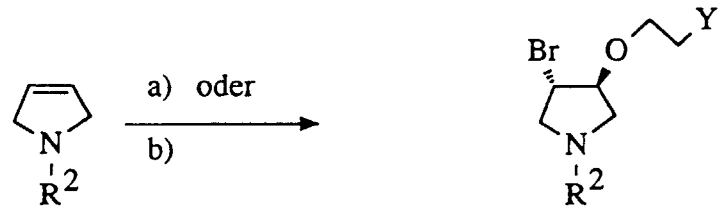


(II) (III)

a) N-Bromsuccinimid,

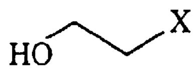

b) $Br_2$, Ethylenoxid

X = OH, Cl, Br,
Y = OH, Cl, Br, $OSO_2R^3$ mit $R^3$ = Methyl, Phenyl, 4-Methylphenyl.

Hierbei werden Verbindungen der Formel (III), in denen Y für OH steht, nach allgemein bekannten Methoden in einen Sulfonsäureester überführt, zum Beispiel mit Methansulfonsäurechlorid oder p-Toluolsulfonsäurechlorid in Gegenwart einer Hilfsbase wie Pyridin, Triethylamin oder Natronlauge.

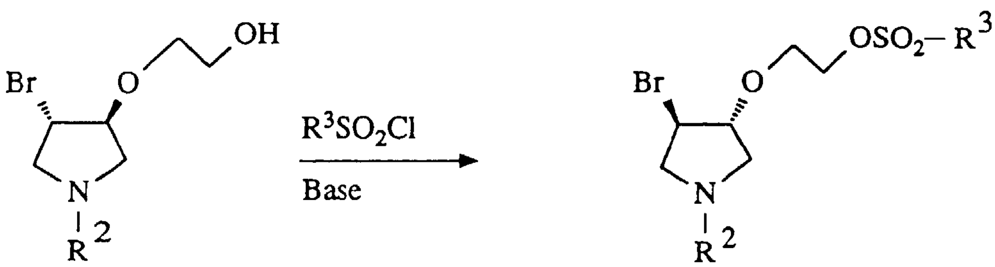


Die als Ausgangsmaterialien benötigten 1-acylierten oder 1-sulfonylierten 2,5-Dihydropyrrole der Formel (II) sind literaturbekannt und können durch Acylierung von 2,5-Dihydropyrrolen (Chem. Pharm. Bull. 17, 980 (1969)) bzw. durch Bromwasserstoff-Abspaltung aus 1-sulfonylierten 3-Brom-tetrahydropyrrolen (Zh. Org. Khim. 3, 1509 (1967)) erhalten werden.

Außerdem können die Verbindungen der Formel (II) nach einem neuen Verfahren durch Umsetzung von Carbonsäureamiden bzw. Sulfonsäureamiden mit cis-1,4-Dichlor-2-buten oder cis-1,4-Dibrom-2-buten und Alkalimetall-hydroxiden und/oder -carbonaten unter Phasentransfer-Katalyse erhalten werden. Als Säureamide können aliphatische und aromatische Carbonsäure- bzw. Sulfonsäureamide eingesetzt werden, wie z.B.: Isobuttersäureamid, Buttersäureamid, Pivalinsäureamid, Phenylessigsäureamid, Benzoesäureamid, 2-Chlor-benzoesäureamid, 4-Chlor-benzoesäureamid, 2,6-Difluor-benzoesäureamid, 4-tert.-Butyl-benzoesäureamid, 4-Methyl-benzolsulfonsäureamid, Methansulfonsäureamid.

Als Säureakzeptoren können Alkalimetall-hydroxide und -carbonate oder deren Mischungen sowohl als Festkörper als auch in Form ihrer wäßrigen Lösungen eingesetzt werden.

Als Phasentransfer-Katalysatoren können alle die für diese Katalyse üblichen Verbindungen eingesetzt werden (siehe z.B. J. Chem. Research (S), 1989, 224 und C.M. Starks, "Phase transfer catalysis", Academic Press, New York, 1978). Als Verdünnungsmittel können alle gegenüber den Reaktanden und dem Säureakzeptor inerten oder nahezu inerten Lösungsmittel verwendet werden, wie z.B. aliphatische und aromatische Kohlenwasserstoffe, halogenierte Aromaten und Ether.

Die Umsetzung von (II) zu (III) nach dem erfindungsgemäßen Verfahren kann mit oder ohne zusätzliches

Lösungsmittel durchgeführt werden. Als Lösungsmittel sind alle inerten Lösungsmittel geeignet. Es kommen in Frage Kohlenwasserstoffe wie Benzol, Toluol oder Xylole, Ether wie Dioxan, Tetrahydrofuran oder t-Butylmethylether, dipolar aprotische Lösungsmittel wie Dimethylformamid, Dimethylsulfoxid oder N-Methylpyrrolidon oder chlorierte Kohlenwasserstoffe wie Methylenchlorid, Chloroform oder Chlorbenzol. Arbeitet man ohne zusätzliches Lösungsmittel, so wird das eingesetzte Ethanolderivat in bis zu zwanzigfachem Überschuß eingesetzt. Die Reaktionstemperatur kann in einem weiten Bereich variiert werden. Man arbeitet bei -20°C bis +50°C, vorzugsweise zwischen 0°C und +30°C.

Im zweiten Schritt des erfindungsgemäßen Verfahrens werden die Verbindungen der Formel (III) mit Ammoniak oder einem primären Amin (IV) zu dem 2-Oxa-5,8-diazabicyclo[4.3.0]nonanderivaten (V) cyclisiert. Dabei kommt es durch $S_N2$-Substitution des Bromatoms zur Ausbildung der cis-Konfiguration

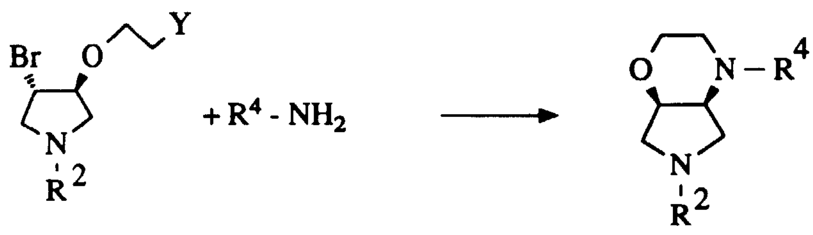

(III) (IV) (V)

$R^4$ = H, $C_1$-$C_3$-Alkyl, Phenyl, Benzyl, (rac, R- oder S-)-1-Phenylethyl.

Die Umsetzung von (III) zu (V) nach dem erfindungsgemäßen Verfahren wird in einem Lösungsmittel durchgeführt. Es können Kohlenwasserstoffe wie Benzol, Toluol, Xylole oder Tetralin, Ether wie Dioxan, Dibutylether, Diethylenglykoldimethylether, Alkohole wie Butanol und Glykolmonomethylether und dipolare aprotische Lösungsmittel wie Dimethylsulfoxid und N-Methylpyrrolidon verwendet werden. Bevorzugt sind Xylole und Tetralin.

Die Reaktionstemperatur kann in einem größeren Bereich variiert werden. Im allgemeinen führt man die Umsetzungen zwischen 80°C und 210°C, vorzugsweise zwischen 110°C und 160°C, durch.

Zur Erzielung höherer Reaktionstemperaturen kann bei Verwendung niedrigsiedender Lösungsmittel oder leicht flüchtiger Amine $R^4$-$NH_2$ unter Druck gearbeitet werden. Man kann bei Drucken von 0,5 bar bis 200 bar, bevorzugt bei 1 bar bis 100 bar, arbeiten.

Um die bei der Reaktion freiwerdenden Halogenwasserstoffe und Sulfonsäuren zu binden, werden Hilfsbasen zugesetzt. Hierzu eignen sich Alkalimetallcarbonate oder -hydrogencarbonate sowie ein Überschuß des eingesetzten Amins $R^4$-$NH_2$ oder andere Amine und Amidine wie Triethylamin, 1,4-Diazabicyclo[2.2.2]octan (Dabco), Diazabicyclo [4.3.0]nonen (DBN) oder Diazabicyclo[6.3.0]undecen (DBU).

In einem weiteren Schritt des erfindungsgemäßen Verfahrens können die Substituenten $R^2$ und $R^4$ der Bicyclen (V), soweit sie Schutzgruppenfunktionen besitzen, wahlweise abgespalten werden. Acylreste werden hydrolytisch entfernt. Zur Hydrolyse eignen sich starke Säuren oder starke Basen. Man verwendet bevorzugt Salzsäure oder Alkalimetall- bzw. Erdalkalimetallhydroxide.

Benzyl- und 1-Phenylethylreste werden hydrogenolytisch entfernt. Man verwendet als Katalysator Palladium, sowohl als Schwamm, als auch auf Trägern wie Aktivkohle, Calciumcarbonat oder Bariumsulfat und Palladiumhydroxid auf Aktivkohle.

Sulfonsäurereste werden reduktiv mit Natrium in flüssigem Ammoniak oder Butanol, mit Natriumnaphthalid oder sauer mit Bromwasserstoff abgespalten.

Die durch Abspalten von Schutzgruppen freien Stickstoffgruppen können gegebenenfalls erneut acyliert werden, so daß aus Verbindungen der Formel (V) Verbindungen, die dem Umfang der Formel (I) entsprechen, hergestellt werden können.

Das erfindungsgemäße Verfahren beinhaltet weiterhin die Trennung der racemischen cis Verbindungen der Struktur (I) in die optischen Antipoden. Diese Racematspaltungen können nach folgenden Verfahren durchgeführt werden:

1) Die racemischen cis- Verbindungen der Formel (I) können, soweit sie basischen Charakter besitzen, mit enantiomerenreinen Säuren, z.B. Carbonsäuren oder Sulfonsäuren wie N-Acetyl-L-glutaminsäure, N-Benzoyl-L-alanin, 3-Brom-campher-9-sulfonsäure, Campher-3-carbonsäure, cis-Camphersäure,

Campher-10-sulfonsäure, O,O'-Dibenzoyl-weinsäure, D- oder L-Weinsäure, Mandelsäure, α-Methoxy-phenylessigsäure, 1-Phenyl-ethansulfonsäure, α-Phenyl-bernsteinsäure zu einem Gemisch der diastereomeren Salze umgesetzt werden, die sich durch fraktionierte Kristallisation zu den enantiomerenreinen Salzen trennen lassen (siehe P.Newmann, Optical Resolution Procedures for Chemical Compounds, Volume 1). Durch Behandlung dieser Salze mit Alkali- oder Erdalkalihydroxiden lassen sich die enantiomerenreinen Amine freisetzen.

Im folgenden Formelschema sei als Beispiel für eine Racematspaltung die Auftrennung von cis-5-Benzyl-2-oxa-5,8-diazabicylco[4.3.0]nonan über die Tartrate und die anschließende Überführung in die enantiomerenreinen cis-2-Oxa-5,8-diazabicyclo[4.3.0]nonane dargestellt:

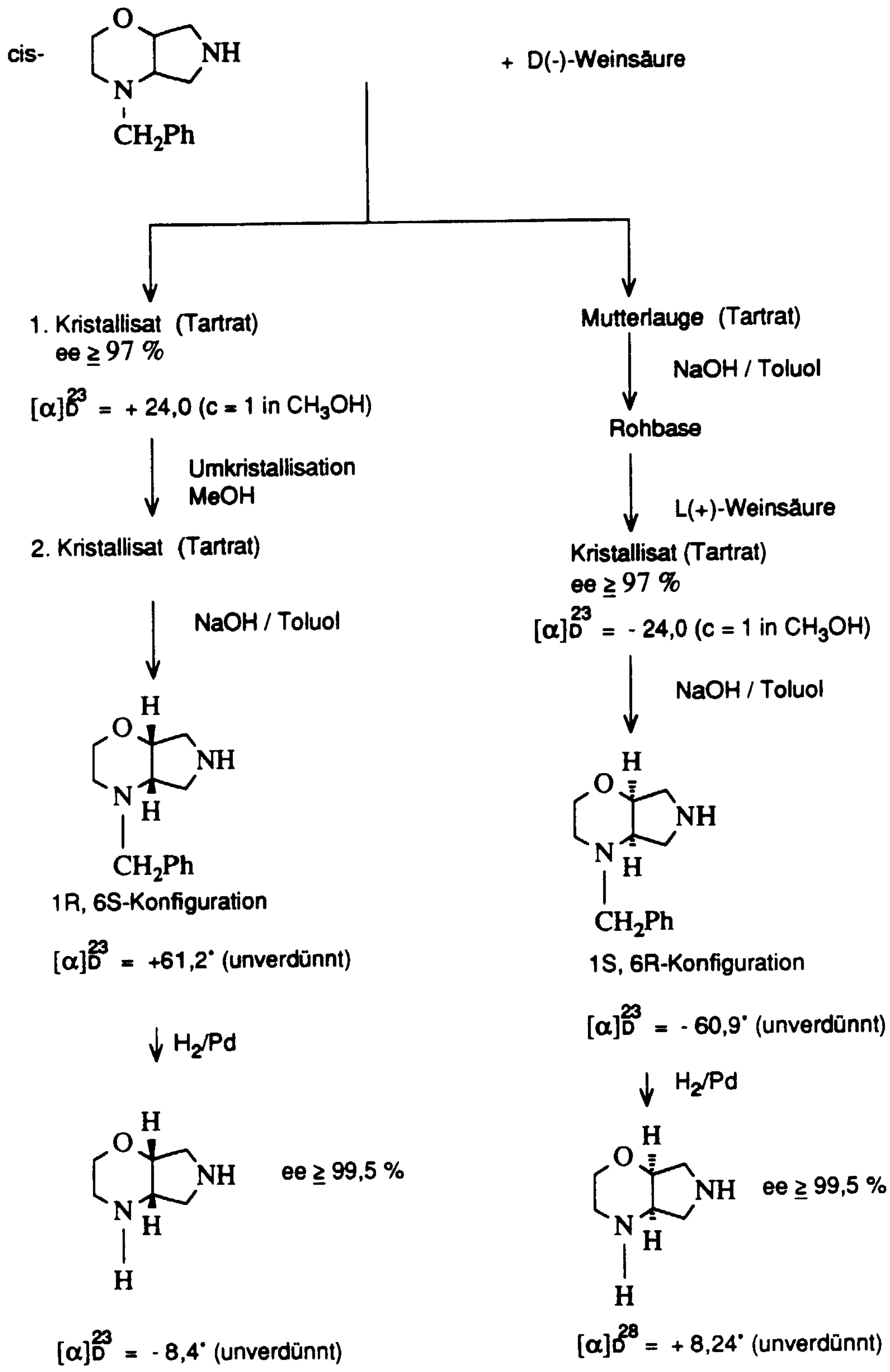


2) Sowohl die racemischen Amine der Formel (I) als auch die racemischen Zwischenstufen der Formel (III) können, gegebenenfalls nach Acylierung, über chirale Trägermaterialien chromatographisch getrennt werden (siehe z.B. G. Blaschke, Angew. Chem. 92, 14 [1980]).

3) Sowohl die racemischen Amine der Formel (I) als auch die racemischen Zwischenprodukte der Formel (III)

können durch chemische Verknüpfung mit chiralen Acylresten in Diastereomerengemische überführt werden, die sich durch Destillation, Kristallisation oder Chromatographie in die enantiomerenreinen Acylderivate trennen lassen, aus denen sich durch Verseifung die enantiomerenreinen Amine isolieren lassen. Beispiele für Reagentien zur Verknüpfung mit chiralen Acylresten sind: α-Methoxy-α-trifluormethylphenylacetylchlorid, Menthylisocyanat, D- oder L-α-Phenyl-ethyl-isocyanat, Chlorameisensäurementhylester, Carnpher-10-sulfonsäurechlorid.

4) Im Verlauf der Synthese der Verbindungen der Formel (I) können statt achiraler auch chirale Schutzgruppen eingeführt werden. Man gelangt auf diese Weise zu Diastereomeren, die sich trennen lassen. Z.B. können für die Cyclisierung (III)-(V) als Aminkomponente die enantiomerenreinen 1-Phenylethylamine eingesetzt werden, wie im folgenden Formelschema gezeigt ist.

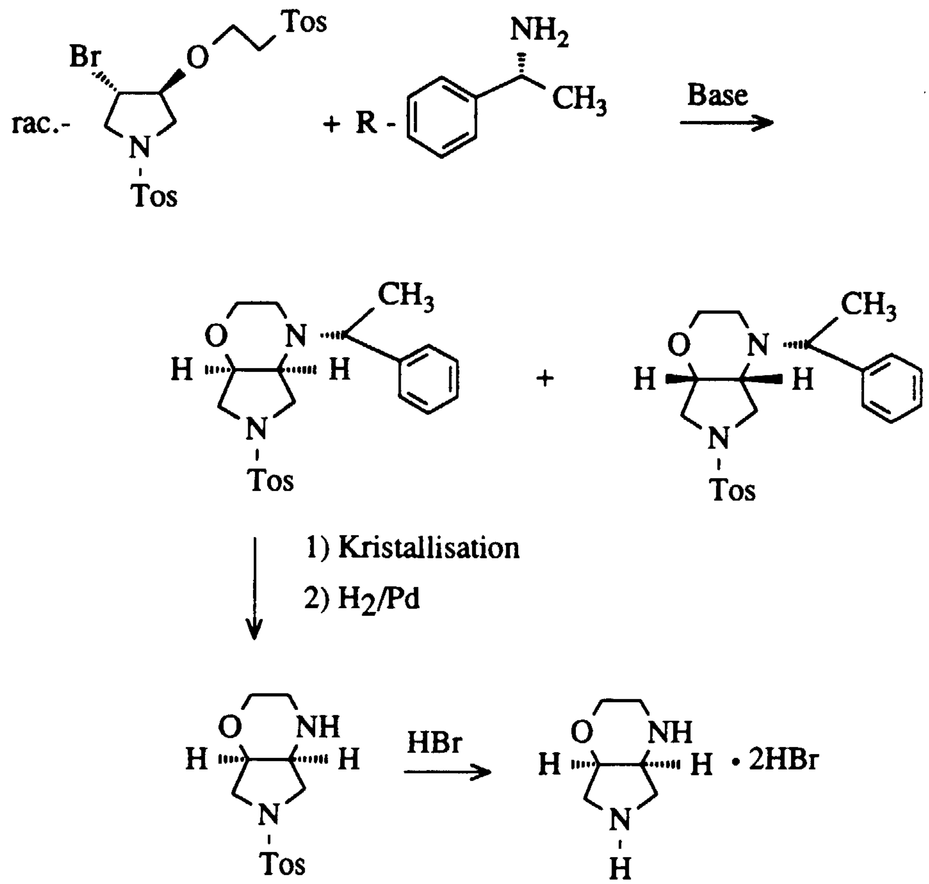


Das erfindungsgemäße Verfahren schließt aber auch die Herstellung enantiomerenreiner Verbindungen der Formel (I) aus enantiomerenreinen Ausgangsmaterialien bzw. unter Verwendung chiraler Auxiliare und Katalysatoren ein. Zur Erläuterung diene die Synthese von 1S,6R-2-Oxa-5,8-diazabicyclo[4.3.0]nonan ausgehend von literaturbekannten S,S-3,4-Dihydroxypyrrolidinderivaten (DOS 3 403 194).

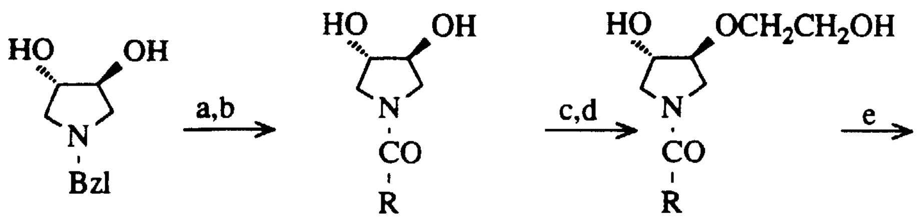

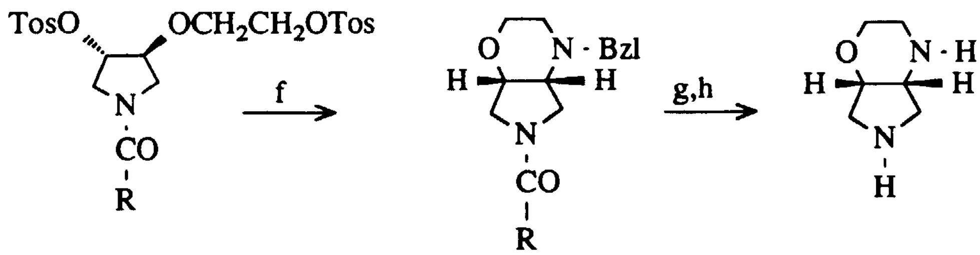


R = zum Beispiel $(CH_3)_3C$-O
a: $H_2$, Pd/A-Kohle
b: Acylierung
c: NaH, $BrCH_2COOC_2H_5$ oder c: $CH_2$=CH-$CH_2$Br, NaH,
d: $LiBH_4$ d: $O_3$, $NaBH_4$,
e: Tosylchlorid, $NEt_3$,
f: Benzylamin, Xylol, Rückfluß
g: Hydrolyse
h: $H_2$, Pd/A-Kohle

Die erfindungsgemäßen Verbindungen stellen wertvolle Zwischenverbindungen für neue Chinolon- und Naphthyridoncarbonsäuren dar, die Gegenstand einer Anmeldung vom gleichen Tage sind.

Sie zeichnen sich durch eine gute Verträglichkeit und durch eine hohe antibakterielle Wirksamkeit aus, die besonders gegenüber grampositiven Keimen stark ausgeprägt ist.

Die nachstehende Tabelle belegt den Vorteil der Verbindung aus Referenzbeispiel 2 im Vergleich zu Ciprofloxacin im Modell der mit Staph. aureus infizierten Maus:

Tabelle:

| Wirksamkeit in der Staph.aureus-Infektion an der Maus (mg/kg) | | |
|---|---|---|
| Substanz | p.o. | s.c. |
| Ciprofloxacin | 80 | 80 |
| Referenzbeispiel 2 | 10 | 10 |

Die folgenden Beispiele erläutern die Erfindung.

Beispiel 1

1-Benzoyl-2,5-dihydro-pyrrol

121 g (1 mol) Benzamid werden in 3 1 Toluol vorgelegt, 200 g Kaliumhydroxid-Pulver unter Rühren eingetragen, 32 g (0,1 mol) Tetrabutylammoniumbromid zugesetzt und auf 40°C erwärmt. Dann werden 243 g (1 mol) 88 %iges cis-1,4-Dibrom-2-buten so zugetropft, daß die Innentemperatur 60°C nicht übersteigt. Es wird noch 5 Stunden bei 50°C nachgerührt, dann auf Wasser gegossen, die organische Phase abgetrennt, mit Wasser gewaschen, getrocknet und eingeengt. Der Rückstand wird im Vakuum destilliert.

| | |
|---|---|
| Ausbeute: | 130 g (75 % der Theorie) |
| Gehalt: | 98 %, gaschromatographisch bestimmt |
| Siedepunkt: | 98 bis 104°C/0,2 mbar. |

Beispiel 2

2,5-Dihydro-1-(4-methyl-phenyl-sulfonyl)-pyrrol

In einem 1,0 l-Rührkessel mit Bodenauslaß-Ventil werden 51,3 g (0,3 mol) p-Toluolsulfonsäureamid, 200 ml (1,2 mol) 6 n Natronlauge, 400 ml Toluol und 4,44 g (15 mmol) Tetrabutyl-ammoniumchlorid vorgelegt und bei einer Innentemperatur von 60°C 37,5 g (0,3 mol) cis-1,4-Dichlor-2-buten unter kräftigem Rühren innerhalb von 20 Minuten

zugetropft, worauf die Innentemperatur um 2°C ansteigt. Es wird noch 1 Stunde bei 60°C und 2 Stunden bei 80°C nachgerührt, dann wird die wäßrige Phase abgetrennt und die organische Phase bei 60°C mit 100 ml 1 n Schwefelsäure und 2 x 200 ml Wasser gewaschen. Die organische Phase wird durch einen mit Toluol befeuchteten Faltenfilter in ein Kristallisationsgefäß filtriert. Beim Abkühlen auf 0°C werden 44,8 g Produkt, Schmelzpunkt: 128 bis 130°C, erhalten. Aus der eingeengten Mutterlauge (ca. 20 g) werden durch Umkristallisieren aus 100 ml Isopropanol oder 60 ml Toluol weitere 12 bis 15 g Produkt, Schmelzpunkt: 125 bis 127°C, erhalten. Ausbeute: 85 bis 90 % der Theorie.

Beispiel 3

cis-5-Benzyl-2-oxa-5,8-diazabicyclo[4.3.0]nonan

a) trans-1-Benzoyl-3-brom-4-(2-hydroxyethoxy)-pyrrolidin

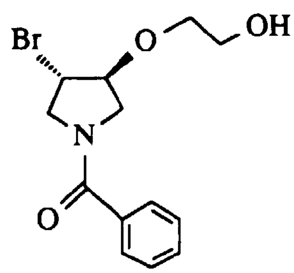

Man löst 95 g (0,55 mol) 1-Benzoyl-3-pyrrolin in 380 g Ethylenglykol und setzt bei Raumtemperatur 101 g (0,57 mol) N-Bromsuccinimid in 5 g-Portionen innerhalb von 2 Stunden hinzu. Man rührt anschließend über Nacht bei Raumtemperatur, gießt auf Wasser, extrahiert mit Methylenchlorid, trocknet über Magnesiumsulfat und engt die Lösung ein. Der Rückstand (188 g) wurde mit Essigsäureethylester an Kieselgel chromatographiert.

| | |
|---|---|
| Ausbeute: | 136,5 g (78 % der Theorie), |
| Gehalt nach GC: | 99 %. |

b) trans-3-Brom-4-(2-hydroxyethoxy)-1-tosylpyrrolidin

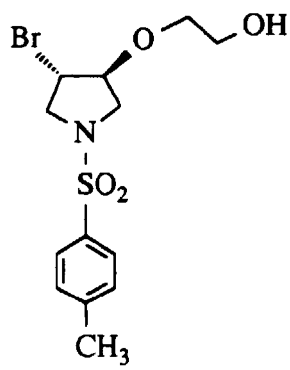

Nach der gleichen Vorschrift wie in Beispiel 3a) wird 1-Tosyl-3-pyrrolin umgesetzt.

| | |
|---|---|
| Ausbeute: | 97,6 % der Theorie, |
| Schmelzpunkt: | 77°C (aus Diisopropylether). |

c) trans-1-Benzoyl-3-brom-4-(2-tosyloxyethoxy)-pyrrolidin

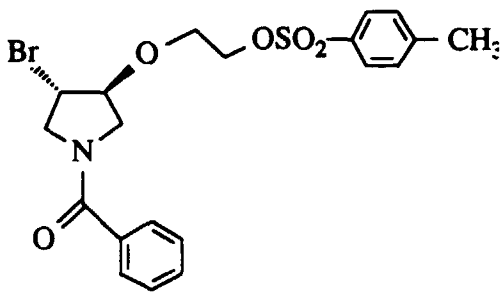

Man löst 92 g (0,239 mol) trans-1-Benzoyl-3-brom-4-(2-hydroxyethoxy)-pyrrolidin, 32 g (0,316 mol) Triethylamin und 1 g 4-Dimethylaminopyridin in 750 ml Toluol und tropft 60 g (0,31 mol) Tosylchlorid in 450 ml Toluol hinzu. Man rührt zwei Tage bei Raumtemperatur, setzt Wasser zu, trennt die wäßrige Phase ab und extrahiert sie mit Toluol. Die Toluollösungen werden mit 10 %iger Salzsäure gewaschen, über Magnesiumsulfat getrocknet, eingeengt, in Essigsäureethylester gelöst und über Kieselgel filtriert. Das Filtrat wird eingeengt.

Ausbeute: 125 g (91 % der Theorie).

Das Dünnschichtchromatogramm zeigt eine einheitliche Verbindung.

d) trans-3-Brom-1-tosyl-4-(2-tosyloxyethoxy)-pyrrolidin

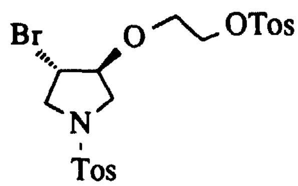

Nach der gleichen Vorschrift wie in Beispiel 3c) wird trans-3-Brom-4-(2-hydroxyethoxy)-1-tosylpyrrolidin umgesetzt.

Ausbeute: 100 % der Theorie
Schmelzpunkt: 144°C (aus Ethanol).

e) cis-8-Benzoyl-5-benzyl-2-oxa-5,8-diazabicyclo[4.3.0]nonan

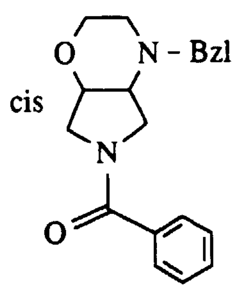

Man erhitzt 124 g (0,265 mol) trans-1-Benzoyl-3-brom-4-(2-tosyloxyethoxy)-pyrrolidin mit 86 g (0,3 mol) Benzylamin in 1,5 1 Xylol über Nacht unter Rückfluß. Man saugt die Salze des Benzylamins ab und engt das Filtrat ein.

Rohausbeute: 91,2 g.

f) cis-5-Benzyl-8-tosyl-2-oxa-5,8-diazabicyclo[4.3.0]nonan

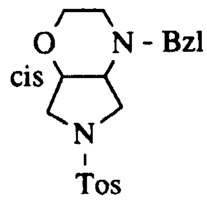

Nach der gleichen Vorschrift wie in Beispiel 3e) wird trans-3-Brom-1-tosyl-4-(2-tosyloxyethoxy)-pyrrolidin umgesetzt.

Ausbeute: 84 % der Theorie
Schmelzpunkt: 122°C (aus Waschbenzin/Butanol 1:1).

g) cis-5-Benzyl-2-oxa-5,8-diazabicyclo[4.3.0]nonan

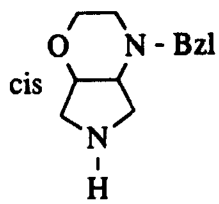

Verfahren A

Man erhitzt 91 g (0,265 mol) cis-8-Benzoyl-5-benzyl-2-oxa-5,8-diazabicyclo[4.3.0]nonan mit 200 ml konzentrierter Salzsäure und 140 ml Wasser über Nacht unter Rückfluß. Nach dem Abkühlen saugt man die Benzoesäure ab, engt auf das halbe Volumen ein, stellt die Lösung mit Kaliumcarbonat alkalisch, extrahiert mit Chloroform, trocknet über Kaliumcarbonat, engt ein und destilliert.

Ausbeute: 30,7 g (48,8 % der Theorie),
Siedepunkt: 134-142°C/0,6 mbar,
Gehalt nach GC: 92 %.

Verfahren B

Man löst 575 g (1,54 mol) cis-5-Benzyl-8-tosyl-2-oxa-5,8-diazabicyclo[4.3.0]nonan in 1,5 1 33 %iger HBr in Essigsäure, setzt 310 g Phenol hinzu und rührt über Nacht bei Raumtemperatur. Man versetzt mit 2 l Diisopropylether, saugt das Dihydrobromid ab, wäscht es mit 0,5 1 Diisopropylether und trocknet an der Luft.

Ausbeute: 505 g (86 % der Theorie).

Man löst das Dihydrobromid in 1 Wasser und versetzt mit 45 %iger Natronlauge bis zur stark alkalischen Reaktion. Man trennt die organische Phase ab, extrahiert die wäßrige Phase dreimal mit insgesamt 1 l tert.-Butylmethylether und einmal mit 300 ml Butanol. Die organischen Lösungen werden über $K_2CO_3$ getrocknet, eingeengt und der Rückstand destilliert.

Ausbeute: 273 g (81 % der Theorie)
Siedepunkt: 130°C/0,07 mbar.

Beispiel 4

Enantiomerentrennung von cis-5-Benzyl-2-oxa-5,8-diazabicyclo[4.3.0]nonan

150,1 g (1 mol) D(-)-Weinsäure werden bei 60 bis 65°C in 700 ml Methanol vorgelegt und 218,3 g (1 mol) cis-5-Benzyl-2-oxa-5,8-diazabicyclo[4.3.0]nonan als Lösung in 300 ml Methanol zugetropft. Dann läßt man langsam auf etwa 49°C erkalten bis die Lösung trübe wird, impft mit in einem Vorversuch erhaltenen Kristallen von IR,6S-

5-Benzyl-2-oxa-5,8-diazabicyclo[4.3.0]nonan-D-tartrat an, rührt 30 Minuten bei dieser Temperatur zur Kristallkeim-Bildung nach und kühlt dann langsam bis auf 0 bis 3°C ab. Nach dem Absaugen wird mit einer auf 0°C gekühlten Mischung aus 200 ml Ethanol und 100 ml Methanol und dann 3 mal mit jeweils 300 ml Ethanol gewaschen und anschließend das Produkt an der Luft getrocknet.

| | |
|---|---|
| Ausbeute: | 160,3 g 1R,6S-5-Benzyl-2-oxa-5,8-diazabicyclo[4.3.0]nonan-tartrat (87 % der Theorie) |
| Schmelzpunkt: | 174,5 bis 176,5°C |
| ee | > 97 % (nach Derivatisierung mit 1-Phenyl-ethylisocyanat und HPLC-Auswertung) |

$[\alpha]_D^{23}$ = +24,0° (c = 1, Methanol)
156,9 g des 1. Kristallisats werden aus 1 500 ml Methanol umkristallisiert.
Ausbeute: 140,0 g (89 % wiedergewonnen)
Schmelzpunkt: 176 bis 177°C
$[\alpha]_D^{23}$ = +25,2° (c = 1, Methanol)

Die methanolische Mutterlauge der 1. Kristallisation wird am Rotationsverdampfer eingeengt. Der sirupöse Rückstand (236 g) wird in 500 ml Wasser gelöst, mit 250 ml 6 n Natronlauge auf pH 12 bis 13 gestellt, 3 mal mit je 350 ml Toluol extrahiert, der Extrakt über Natriumcarbonat getrocknet und im Vakuum eingeengt. Der Rückstand, 113,1 g eines braunen Öls, das nach gaschromatographischer Untersuchung 97 % cis-5-Benzyl-2-oxa-5,8-diazabicyclo [4.3.0]nonan enthält, wird ohne Aufreinigung zur Herstellung des 1S,6R-Enantiomeren eingesetzt.

113,1 g (0,518 mol) rohes angereichertes IS,6R-5-Benzyl-2-oxa-5,8-diazabicyclo[4.3.0]nonan werden in 155 ml Methanol gelöst und zu einer siedenden Lösung von 77,8 g (0,518 mol) L(+)-Weinsäure in 363 ml Methanol getropft. Schon während des Zutropfens bildet sich allmählich ein Kristallbrei. Es wird 1 Stunde bei 60°C nachgerührt und dann langsam innerhalb von 2 Stunden auf 0°C abgekühlt. Die Kristalle werden abgesaugt und mit einer auf 0°C gekühlten 2:1-Mischung aus Ethanol und Methanol und anschließend 3 mal mit Ethanol gewaschen. Dann wird an der Luft getrocknet.

| | |
|---|---|
| Ausbeute: | 145,5 g 1S,6R-5-Benzyl-2-oxa-5,8-diazabicyclo[4.3.0]nonan-L-tartrat (79 % der Theorie) |
| Schmelzpunkt: | 174,5 bis 176,5°C |
| ee | > 97 % (nach Derivatisierung mit 1-Phenyl-ethylisocyanat und HPLC-Auswertung) |

$[\alpha]_D^{23}$ = -24,0° (c = 1, Methanol)

Freisetzung der enantiomerenreinen Basen:
144 g (0,39 mol) 1S,6R-5-Benzyl-2-oxa-5,8-diazabicyclo[4.3.0]nonan-tartrat werden in 250 ml Wasser gelöst und 175 ml (1,05 mol) 6 n Natronlauge zugesetzt. Das abgeschiedene Öl wird in 500 ml Toluol aufgenommen, die organische Phase abgetrennt und die wäßrige Phase noch 3 mal mit jeweils 250 ml Toluol extrahiert. Die vereinigten organischen Phasen werden über Natriumcarbonat getrocknet, filtriert und am Rotationsverdampfer eingeengt. Der Rückstand wird über eine 20 cm Vigreuxkolonne im Hochvakuum destilliert.

| | |
|---|---|
| Ausbeute: | 81,6 g (96 % der Theorie) 1S,6R-5-Benzyl-2-oxa-5,8-diazabicyclo[4.3.0]nonan |
| Siedepunkt: | 120 bis 139°C/0,04 bis 0,07 mbar |
| Gehalt: | 100 % gaschromatographisch bestimmt |

Dichte: = 1,113 g/m1
$[\alpha]_D^{23}$ = -60,9° (unverdünnt)
Destillationsrückstand: 0,12 g
In gleicher Weise werden aus 139,2 g (0,376 mol) IR,6S-5-Benzyl-2-oxa-5,8-diazabicyclo[4.3.0]nonan-tartrat 76,0 g (93 % der Theorie) IR,6S-5-Benzyl-2-oxa-5,8-diazabicyclo[4.3.0]nonan erhalten.
$[\alpha]_D^{23}$ = +61,2° (unverdünnt).

Die für das cis-5-Benzyl-2-oxa-5,8-diazabicyclo[4.3.0]nonan beschriebene Enantiomerentrennung kann analog auch mit trans-8-Benzyl-2-oxa-5,8-diazabicyclo[4.3.0]nonan zu R,R- und S,S-8-Benzyl-2-oxa-5,8-diazabicyclo [4.3.0]nonan durchgeführt werden.

<u>Beispiel 5</u>

a) 3S,4S-4-Allyloxy-3-hydroxypyrrolidin-1-carbonsäure-tert.-butylester

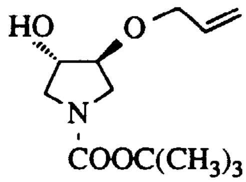

Man legt 16,5 g (0,55 mol) 80 %iges NaH in 500 ml absolutem Dioxan vor und tropft bei 60°C eine Lösung von 107,5 g (0,53 mol) S,S-3,4-Dihydroxypyrrolidin-1-carbonsäure-tert.-butylester (DOS 3 403 194) in absolutem Dioxan heiß gelöst hinzu. Man rührt eine Stunde bei 60°C und tropft dann 64 g (0,53 mol) Allylbromid hinzu. Anschließend rührt man drei Stunden bei 60°C. Man engt ein und löst den Rückstand in 200 ml Wasser und 600 ml Methanol. Man extrahiert dreimal mit je 200 ml Pentan, zieht das Methanol am Rotationsverdampfer ab, verdünnt mit 200 ml Wasser und extrahiert mit Methylenchlorid. Die Methylenchloridlösung wird über $MgSO_4$ getrocknet, eingeengt und in tert.-Butylmethylether (200 ml) gelöst. Über Nacht kristallisierten hieraus 9 g Edukt (44 mmol). Die Etherlösung wird eingeengt und destilliert.

Ausbeute: 83 g (80 % der Theorie bezogen auf wiedergewonnenes Edukt und Diallylether)
Siedepunkt: 149°C/0,7 mbar bis 159°C/0,9 mbar.

Das Destillat enthält 5 % des Eduktes und 4 % des Diallylethers.
Der Pentanextrakt lieferte 17 g eines Gemisches aus 15 % gewünschtem Produkt und 84 % des Diallylethers.
$[\alpha]_D^{23}$ = -10,5° (c = 1, Methanol).

b) 3S,4S-3-Hydroxy-4-(2-hydroxyethoxy)-pyrrolidin-1-carbonsäure-tert.-butylester

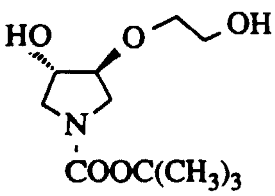

Man löst 64 g (0,24 mol, 91 %ig) 3S,4S-4-Allyloxy-3-hydroxypyrrolidin-1-carbonsäure-tert.-butylester in 250 ml Methanol, kühlt auf 0°C und leitet Ozon durch die Lösung, bis eine nachgeschaltete Waschflasche mit Kaliumiodidlösung das Auftreten von Ozon und damit vollständige Umsetzung anzeigt. Ozonreste werden durch einen Stickstoffstrom ausgetragen, dann wird bei 0°C das entstandene Ozonid mit 18 g Natriumborhydrid reduziert, welches in 1 g-Portionen zugesetzt wird. Anschließend rührt man über Nacht bei Raumtemperatur, engt den Ansatz ein, verdünnt mit Wasser, versetzt mit 20 g Kaliumcarbonat und extrahiert fünfmal mit je 100 ml Methylenchlorid. Man trocknet die organischen Lösungen über Magnesiumsulfat und engt ein.

Ausbeute: 65,8 g (100 % der Theorie).
Gehalt: 91 % (gaschromatographisch bestimmt).

$[\alpha]_D^{20}$ = -15,2° (c = 0,97, Methanol).

c) 3S,4S-3-Tosyloxy-4-(2-tosyloxyethoxy)-pyrrolidin-1-carbonsäure-tert.-butylester

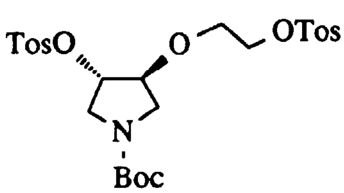

Man legt 2,7 g (10 mmol, 91 %ig) 3S,4S-3-Hydroxy-4-(2-hydroxyethoxy)-pyrrolidin-1-carbonsäure-ten.-butylester in 30 ml Methylenchlorid vor, setzt 6 ml 45 %ige Natronlauge und 0,1 g Benzyltriethylammoniumchlorid hinzu und tropft dann unter Kühlung eine Lösung von 3,86 g (20 mmol) Tosylchlorid in 10 ml Methylenchlorid hinzu. Man rührt anschließend noch eine Stunde bei Raumtemperatur, gießt auf 20 ml Wasser, trennt die organische Phase ab und extrahiert die wäßrige Phase mit Methylenchlorid. Die organischen Phasen werden über Magnesiumsulfat getrocknet und eingeengt.

Ausbeute: 5 g (90 % der Theorie).

Das Produkt ist dünnschichtchromatografisch einheitlich.

d) 1S,6R-5-Benzyl-2-oxa-5,8-diazabicyclo[4.3.0]nonan-8-carbonsäure-tert.-butylester

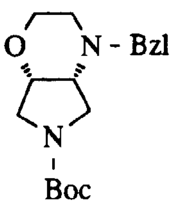

Man erhitzt 87 g (156 mmol) 3S,4S-3-Tosyloxy-4(2-tosyloxyethoxy)pyrrolidin-1-carbonsäure-tert.-butylester mit 58 g (0,54 mol) Benzylamin in 11 Xylol über Nacht unter Rückfluß. Man kühlt ab, saugt ausgefallene Salze des Benzylamins ab und engt den Rückstand ein.

Ausbeute: 43 g (58 % der Theorie).
Gehalt: 67 % (gaschromatographisch bestimmt).

e) 1S,6R-5-Benzyl-2-oxa-5,8-diazabicyclo[4.3.0]nonan

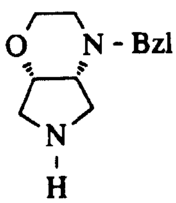

Man erhitzt 43 g (90 mmol) 1S,6R-5-Benzyl-2-oxa-5,8-diazabicyclo[4.3.0]nonan-8-carbonsäure-tert.-butylester in 35 ml konzentrierter Salzsäure und 35 ml Wasser bis zum Ende der Kohlendioxidentwicklung unter Rückfluß. Man stellt mit Kaliumcarbonat alkalisch, extrahiert mit Chloroform, trocknet die organischen Lösungen über MgSO4, engt ein und destilliert zweimal über eine 20 cm-Vigreux-Kolonne.

Ausbeute: 11,1 g (55 % der Theorie)
Siedepunkt: 108 bis 115°C/0,07 mbar

$[\alpha]_D^{26}$ = -58,3° (unverdünnt).

Beispiel 6

a) 3R,4R-4-Allyloxy-3-hydroxypyrrolidin-1-carbonsäure-tert.-butylester

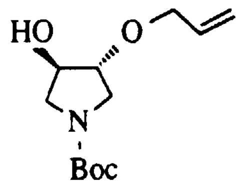

Umsetzung analog Beispiel 5a) aus R,R-3,4-Dihydroxypyrrolidin-1-carbonsäure-tert.-butylester

Siedepunkt: 145°C/0,1 mbar

$[\alpha]_D^{23}$= +9,5° (c = 1,0, Methanol)
Gehalt: 95 % (gaschromatographisch bestimmt).

b) 3R,4R-3-Hydroxy-4-(2-hydroxyethoxy)-pyrrolidin-1-carbonsäure-tert.-butylester

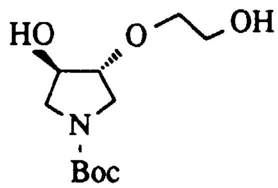

Umsetzung analog Beispiel 5b) mit 3R,4R-4-Allyloxy-3-hydroxypyrrolidin-1-carbonsäure-tert.-butylester

Ausbeute: 99 % der Theorie (0,175 molarer Ansatz)

$[\alpha]_D^{20}$ = +16,5° (c = 0,94, Methanol)

c) 3R,4R-3-Tosyloxy-4-(2-tosyloxyethoxy)-pyrrolidin-1-carbonsäure-tert.-butylester

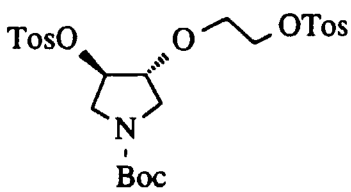

Umsetzung analog Beispiel 5c) mit 3R,4R-3-Hydroxy-4-(2-hydroxyethoxy)pyrrolidin-1-carbonsäure-tert.-butylester

Ausbeute: quantitativ (0,11 molarer Ansatz).

d) 1R,6S-5-Benzyl-2-oxa-5,8-diazabicyclo[4.3.0]nonan-8-carbonsäure-tert.-butylester

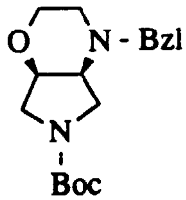

Umsetzung analog Beispiel 5d) mit 3R,4R-3-Tosyloxy4-(2-tosyloxyethoxy)-pyrrolidin-1-carbonsäure-tert.-butylester

Ausbeute: 40 % der Theorie (0,1 molarer Ansatz).

e) 1R,6S-5-Benzyl-2-oxa-5,8-diazabicyclo[4.3.0]nonan

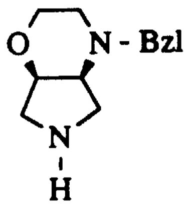

Umsetzung analog Beispiel 5e) mit 1R,6S-5-Benzyl-2-oxa-5,8-diazabicyclo[4.3.0]nonan-8-carbonsäure-tert.-butylester

Ausbeute: 63 % der Theorie (40 mmolarer Ansatz)
Siedepunkt: 120°C/0,06 mbar
Gehalt: 95 % (gaschromatographisch bestimmt).

$[\alpha]_D^{28}$ = +58,5° (unverdünnt).

Beispiel 7

a) 1S,6R-2-Oxa-5,8-diazabicyclo[4.3.0]nonan-Dihydrochlorid

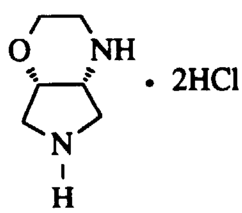

Man hydriert 7,5 g (34,4 mmol) IS,6R-5-Benzyl-2-oxa-5,8-diazabicyclo[4.3.0]nonan in 200 ml Ethanol unter Zusatz von 7 ml konzentrierter Salzsäure an 1 g Palladium-Aktivkohle (10 % Pd) bei 100°C und 100 bar. Man saugt den Katalysator ab und wäscht ihn mehrfach mit Wasser. Das wäßrige Filtrat wird eingeengt, worauf der Rückstand kristallisiert. Die Kristalle werden gründlich mit Ethanol verrieben, abgesaugt und an der Luft getrocknet.

Ausbeute: 4,6 g (66,5 % der Theorie)
Schmelzpunkt: 233 bis 235°C.

b) 1S,6R-2-Oxa-5,8-diazabicyclo[4.3.0]nonan

Man hydriert 59 g (0,27 mol) IS,6R-5-Benzyl-2-oxa-5,8-diazabicyclo[4.3.0]nonan in 500 ml Ethanol an 5 g Palladium-Aktivkohle (10 % Pd) bei 120°C und 120 bar. Der Katalysator wird abgesaugt, das Filtrat eingeengt und der Rückstand destilliert.

Ausbeute: 32,9 g (95 % der Theorie)
Siedepunkt: 65°C/0,03 mbar

$[\alpha]_D^{28}$ : +8,2° (unverdünnt).
Bestimmung des ee-Wertes mit Mosher-Reagenz:

Man löst 0,1 mmol des Amins in 1,5 ml Toluol, setzt 0,3 ml 1 n Natronlauge, 0,3 ml Wasser und 0,25 ml einer 1 n Lösung von 3,3,3-Trifluor-2-methoxy-2-phenylpropansäurechlorid (Mosher-Reagenz) in Toluol hinzu und rührt 30 Minuten bei Raumtemperatur. Die Toluollösung wird abpipettiert und direkt zur gaschromatographischen Analyse verwendet. Das Gaschromatogramm zeigt nur ein detektierbares Enantiomeres (ee ≧ 99,5 %), während das Racemat zwei basisliniengetrennte Peaks für die Mosherderivate beider Enantiomerer zeigt.

Beispiel 8

a) 1R,6S-2-Oxa-5,8-diazabicyclo[4.3.0]nonan-Dihydrochlorid

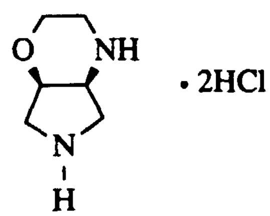

Die Umsetzung erfolgt analog Beispiel 7a) mit IR,6S-5-Benzyl-oxa-5,8-diazabicyclo[4.3.0]nonan.

Ausbeute: 77 % der Theorie (23,8 mmolarer Ansatz)
Schmelzpunkt: 230 bis 232°C.

b) 1R,6S-2-Oxa-5,8-diazabicyclo[4.3.0]nonan

Die Umsetzung erfolgt analog Beispiel 7b) mit IR,6S-5-Benzyl-2-oxa-5,8-diazabicyclo[4.3.0]nonan.

Ausbeute: 93,3 % der Theorie (1,58 molarer Ansatz)
Siedepunkt: 63 bis 65°C/0,03 mbar

$[\alpha]_D^{23}$: -8,4° (unverdünnt)
ee-Wert: ≧ 99,5 % (durch Derivatisierung mit Mosher-Reagenz).

1R,6R- bzw. 1S,6S-2-Oxa-5,8-diazabicyclo[4.3.0]-nonan können analog erhalten werden.

Beispiel 9

1R,6S-2-Oxa-5,8-diazabicyclo[4.3.0]nonan-Dihydrobromid

a) 1R,6S-5-(1R-Phenylethyl)-8-tosyl-2-oxa-5,8-diazabicyclo[4.3.0]nonan

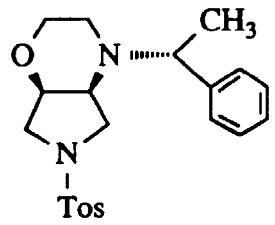

Man erhitzt 101,8 g (0,196 mol) trans-3-Brom-1-tosyl4-(2-tosyloxyethoxy)-pyrrolidin und 72 g (0,584 mol) R-(+)-1-Phenylethylamin in 900 ml Xylol über Nacht unter Rückfluß. Man wäscht die abgekühlte Lösung mit 2 n Natronlauge, trocknet über Kaliumcarbonat, entfernt das Trockenmittel und engt die Lösung ein. Beim Abkühlen scheiden sich aus dem Rückstand Kristalle ab, die abgesaugt und aus einem Gemisch aus 750 ml Waschbenzin und 200 ml n-Butanol umkristallisiert wurden.

| | |
|---|---|
| Ausbeute: | 15 g (39,6 % der Theorie an optisch reinem Material) |
| Schmelzpunkt: | 188°C |

$[\alpha]_D^{28}$: +103,7° (c = 1,$CHCl_3$).

b) 1R,6S-8-Tosyl-2-oxa-5,8-diazabicyclo[4.3.0]nonan

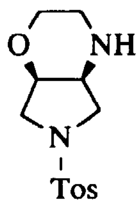

Man hydriert 13 g (33,6 mmol) IR,6S-5-(IR-Phenylethyl)-8-tosyl-2-oxa-5,8-diazabicyclo[4.3.0]nonan in 200 ml Ethanol an 2,5 g Palladium-A-Kohle (10 % Pd) bei 100°C und 100 bar. Man saugt den Katalysator ab, engt das Filtrat ein und kristallisiert aus 30 ml Toluol um.

| | |
|---|---|
| Ausbeute: | 7,5 g (79 % der Theorie) |
| Schmelzpunkt: | 160 bis 161°C |

$[\alpha]_D^{23}$ : +17,5° (c = 1,21, $CHCl_3$).

c) 1R,6S-2-Oxa-5,8-diazabicyclo[4.3.0]nonan-Dihydrobromid

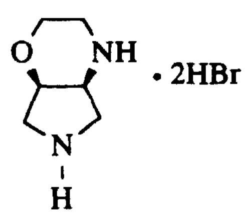

Man löst 7 g (24,8 mmol) IR,6S-8-Tosyl-2-oxa-5,8-diazabicyclo[4.3.0]nonan in 25 ml 33 %iger Bromwasserstofflösung in Eisessig, setzt 5 g Phenol hinzu und rührt über Nacht bei Raumtemperatur. Man verdünnt mit Diisopropylether, saugt das auskristallisierte Salz ab und trocknet es an der Luft.

Ausbeute: 5,5 g.

Die Derivatisierung mit Mosher-Reagenz und gaschromatographischer Analyse (siehe Beispiel 5) zeigt nur ein detektierbares Enantiomeres (ee ≧ 99,5 %).

Referenzbeispiel 1

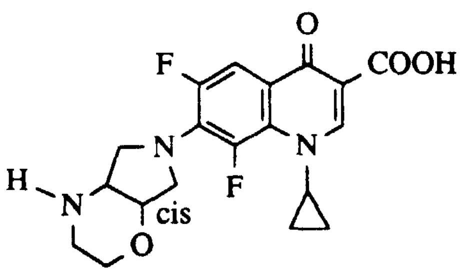

A. 1-Cyclopropyl-6,8-difluor-1,4-dihydro-7-(cis-2-oxa-5,8-diazabicyclo[4.3.0]non-8-yl)-4-oxo-3-chinolincarbonsäure:

1,43 g (5 mmol) 1-Cyclopropyl-6,7,8-trifluor-1,4-dihydro-4-oxo-3-chinolincarbonsäure werden in einer Mischung aus 15 ml Acetonitril/75 ml Dimethylformamid in Gegenwart von 0,67 g (6 mmol) 1,4-Diazabicyclo[2.2.2]octan mit 0,74 g (5,4 mmol) 93 %igem cis-2-Oxa-5,8-diazabicyclo[4.3.0]nonan 1 Stunde unter Rückfluß erhitzt. Die Suspension wird eingeengt, der Rückstand mit Wasser verrührt, der Niederschlag abgesaugt und bei 80°C im Vakuum getrocknet.

Ausbeute: 1,67 g (85,4 % der Theorie),
Schmelzpunkt: 210-212°C (unter Zersetzung).

B. 1-Cyclopropyl-6,8-difluor-1,4-dihydro-7-(cis-2-oxa-5,8-diazabicyclo[4.3.0]non-8-yl)4-oxo-3-chinolincarbonsäure-Hydrochlorid:

1,6 g (4 mmol) des Produkts aus Stufe A werden in 120 ml halbkonzentrierter Salzsäure bei 60°C gelöst, die Lösung eingeengt, der Rückstand mit Ethanol gewaschen und der Niederschlag abgesaugt und bei 90°C im Vakuum getrocknet.

Ausbeute: 1,57 g,
Schmelzpunkt: 300-303°C (unter Zersetzung),
Gehalt (HPLC): 97 %ig.

C. Analog Referenzbeispiel 1A erhält man mit IR,6S-2-Oxa-5,8-diazabicyclo[4.3.0]nonan 1-Cyclopropyl-6,8-difluor-1,4-dihydro-7-(1R,6S-2-oxa-5,8-diazabicyclo[4.3.0]non-8-yl)-4-oxo-3-chinolincarbonsäure vom Schmelzpunkt 204-206°C (unter Zersetzung).

D. Analog Referenzbeispiel 1B erhält man mit dem Betain aus Referenzbeispiel 1C 1-Cyclopropyl-6,8-difluor-1,4-dihydro-7-(1R,6S-2-oxa-5,8-diazabicyclo[4.3.0]non-8-yl)-4-oxo-3-chinolincarbonsäure-Hydrochlorid vom Schmelzpunkt 324-325°C (unter Zersetzung).
$[\alpha]_D^{24}$ : -241° (c =0,59, $H_2O$).

E. Analog Referenzbeispiel 1A erhält man mit IS,6R-2-Oxa-5,8-diazabicyclo[4.3.0]nonan 1-Cyclopropyl-6,8-difluor-1,4-dihydro-7-(1S,6R-2-oxa-5,8-diazabicyclo[4.3.0]non-8-yl)4-oxo-3-chinolincarbonsäure vom Schmelzpunkt 204-206°C (unter Zersetzung).
$[\alpha]_D^{25}$ : +248° (c =0,57, DMF).

F. Analog Referenzbeispiel 1B erhält man mit dem Betain aus Referenzbeispiel 1E 1-Cyclopropyl-6,8-difluor-1,4-dihydro-7-(1S,6R-2-oxa-5,8-diazabicyclo[4.3.0]non-8-yl)-4-oxo-3-chinolincarbonsäure-Hydrochlorid vom Schmelzpunkt 323°C (unter Zersetzung).
$[\alpha]_D^{26}$ : +238° (c =0,5, $H_2O$).

Referenzbeispiel 2

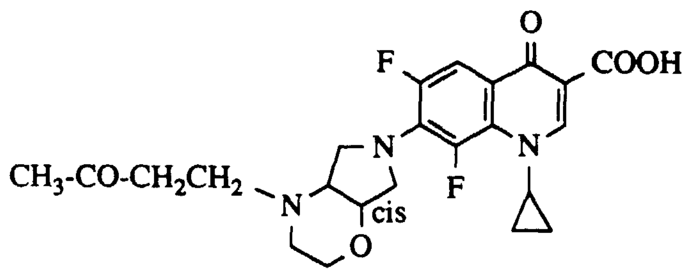

1,56 g (4 mmol) des Produkts aus Referenzbeispiel 1A werden in 50 ml Ethanol mit 1,8 g (25,6 mmol) Methylvinylketon 3 Stunden unter Rückfluß erhitzt. Die Suspension wird bei 70°C/12 mbar eingeengt, der Rückstand mit Wasser verrührt und aus Glykolmonomethylether umkristallisiert.

Ausbeute: 1,33 g (72 % der Theorie) 1-Cyclopropyl-6,8-difluor-1,4-dihydro-4-oxo-7-(cis-5-[3-oxo-1-butyl]-2-oxa-5,8-diazabicyclo[4.3.0]non-8-yl)-3-chinolincarbonsäure,
Schmelzpunkt: 188-189°C (unter Zersetzung).

**Patentansprüche**

1. Verfahren zur Herstellung von enantiomereinreinen Cis-Verbindungen der Formel (I)

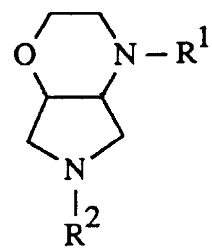

(I)

in welcher

$R^1$ für H, $C_1$-$C_3$-Alkyl, Phenyl, Benzyl, 1-Phenylethyl, $C_1$-$C_3$-Alkanoyl, $C_1$-$C_4$-Alkoxycarbonyl steht

und

$R^2$ für H, Benzyl, $C_1$-$C_5$-Alkanoyl, gegebenenfalls ein- oder zweifach durch Halogen oder $C_1$-$C_4$-Alkyl substituiertes Benzoyl, $C_1$-$C_4$-Alkoxycarbonyl, Methansulfonyl, Benzolsulfonyl und Toluolsulfonyl steht,

dadurch gekennzeichnet, daß 2,5-Dihydropyrrolderivate der Formel (II)

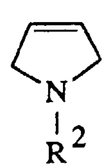

(II)

in welcher $R^2$ die oben angegebene Bedeutung mit Ausnahme von Wasserstoff hat mit Verbindungen der Formel (VIa)

$$Y\text{-}CH_2\text{-}CH_2\text{-}OR \qquad (VIa)$$

worin

Y Brom, Chlor, Hydroxy oder -OSO-$R^3$, worin $R^3$ Methyl, Phenyl oder Methylphenyl bedeutet

und

R Wasserstoff bedeutet

oder

Y und R gemeinsam eine Einfachbindung bedeuten

in Gegenwart von Brom oder N-Bromsuccinimid zu racemische trans- Verbindungen der Formel (III) umgesetzt werden

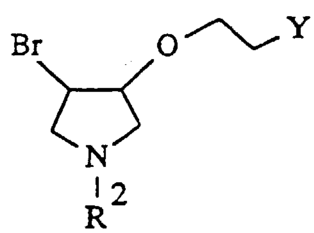

(III)

worin $R^2$ und Y die oben angegebene Bedeutung haben, wobei aus Verbindungen der Formel (III), in denen Y Hydroxy bedeutet, gegebenenfalls die Derivate der Formel (III), in denen Y die übrigen oben genannten Bedeutungen hat, nach allgemein bekannten Methoden erhalten werden können,
die so erhaltenen Verbindungen der Formel (III) entweder mit Aminen der Formel (IV)

$$R^4\text{-}NH_2 \qquad (IV)$$

in welcher

$R^4$ für Wasserstoff, $C_1$-$C_3$-Alkyl, Phenyl, Benzyl oder 1-Phenylethyl steht,

zu racemischen cis- Verbindungen der Formel (V) cyclisiert

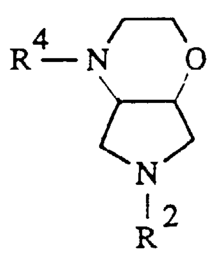

(V)

in welcher
$R^2$ und $R^4$ die oben angegebenen Bedeutungen besitzen,
und anschließend gegebenenfalls als Schutzgruppe dienende Reste $R^2$ und/oder $R^4$ mit geeigneten Methoden

wie Reduktion und/oder Hydro(geno)-lyse entfernt und die so erhaltenen sekundären Stickstoffatome erneut mit Verbindungen $R^1$-X und $R^2$-X, wobei X eine Abgangsgruppe bedeutet, umsetzt und die Racemate der Verbindungen der Formel (I) nach an sich bekannten Methoden spaltet und die enantiomeren Verbindungen der Formeln (Ia - Ib) isoliert

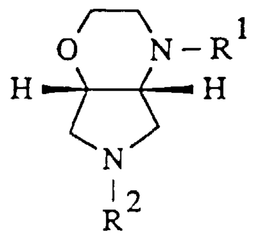

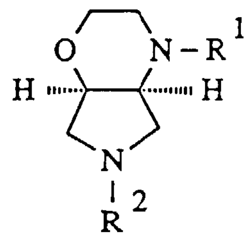

(Ia) (Ib)

oder racemische trans- Verbindungen der Formel (III) mit enantiomerenreinem R- oder S1-Phenylethylamin zu Gemischen von jeweils zwei diastereomeren Verbindungen der Formel (VII), worin $R^2$ die oben genannte Bedeutung hat, umsetzt

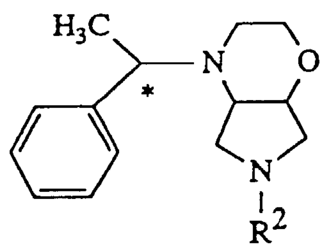

(VII)

die Gemische auf an sich bekannte Weise zu den enantiomerenreinen Verbindungen auftrennt, gegebenenfalls den Phenylethyl-Substituenten hydrogenolytisch abspaltet und erneut mit Verbindungen $R^2$-X, worin X eine Abgangsgruppe bedeutet, umsetzt und die enantiomerenreinen Verbindungen der Formel (Ia-Ib) isoliert
oder indem man S,S- oder R,R-Dihydroxypyrrolidinderivate der Formel (VIIIa) oder (VIIIb)

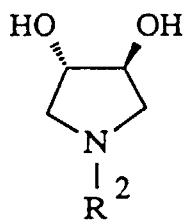

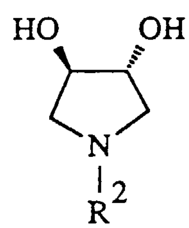

(VIIIa) (VIIIb)

in denen $R^2$ die oben angegebene Bedeutung hat, aber nicht Wasserstoff sein kann, mit Allylbromid oder Allylchlorid in Gegenwart einer Base zu Verbindungen der Formel (IXa) bzw. (IXb) umsetzt

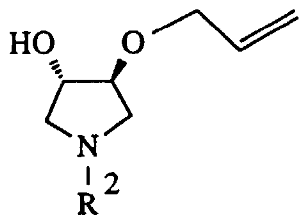

(IXa)

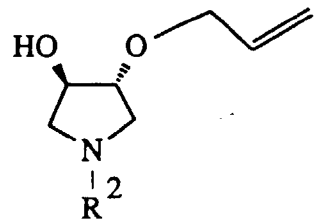

(IXb)

die durch Ozonolyse und anschließende Reduktion zur Verbindungen der Formel (Xa) beziehungsweise (Xb) abgebaut werden

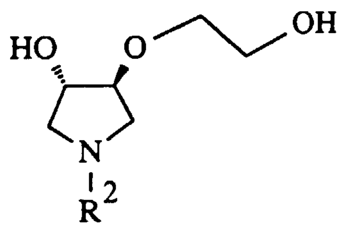

(Xa)

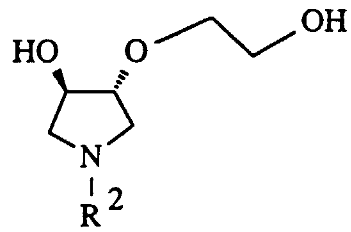

(Xb)

welche durch Acylierung mit Sulfonsäurechloriden $R^3$-$SO_2Cl$ zu Sulfonsäureestern der Formel (XIa) bzw. (XIb) umgesetzt werden, wobei $R^3$ die oben angegebene Bedeutung besitzt,

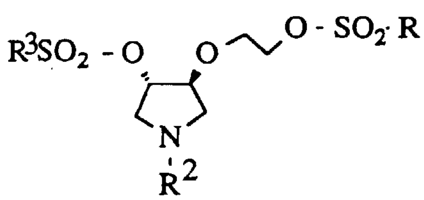

(XIa)

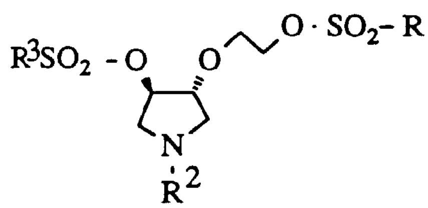

(XIb)

welche durch die Umsetzung mit Aminen der Formel (IV),

$$R^4\text{-}NH_2 \qquad (IV)$$

in welcher $R^4$ die oben angegebene Bedeutung besitzt, zu Verbindungen der Formel (Va) bzw. (Vb) cyclisiert werden,

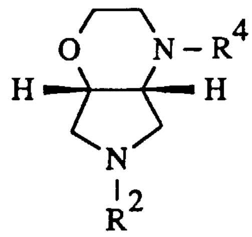
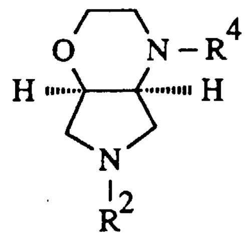

(Va) (Vb)

und anschließend gegebenenfalls als Schutzgruppe dienende Reste $R^2$ und/oder $R^4$ mit geeigneten Methoden wie Reduktion und/oder Hydro(geno)-lyse entfernt und die so erhaltenen sekundären Stickstoffatome erneut mit Verbindungen $R^1$-X und $R^2$-X, wobei X eine Abgangsgruppe bedeutet, umsetzt und die erhaltenen Racemate der Verbindungen der Formel (I) nach an sich bekannten Methoden spaltet und die enantiomeren Verbindungen der Formeln (Ia-Ib) isoliert.

**2.** Verfahren nach Anspruch 1, dadurch gekennzeichnet, daß in Formel (I)

$R^1$ für H, Methyl, Benzyl, 1-Phenylethyl, Acetyl, Ethoxycarbonyl und t-Butoxycarbonyl steht

und

$R^2$ für H, Benzyl, Acetyl, Benzoyl, Ethoxycarbonyl, t-Butoxycarbonyl, Methansulfonyl und p-Toluolsulfonyl steht.

**3.** Verfahren nach Ansprüchen 1 und 2, dadurch gekennzeichnet, daß die Umsetzung der Verbindungen der Formel (III) mit Aminen der Formel (IV) in Xylol oder Tetralin als Lösungsmittel durchgeführt wird.

**4.** Enantiomerenreine cis- Verbindungen der Formel (I)

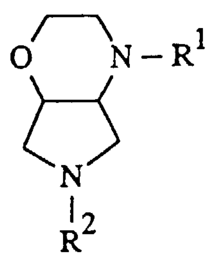

(I)

wobei

$R^1$ für H, $C_1$-$C_3$-Alkyl, Phenyl, Benzyl, 1-Phenylethyl, $C_1$-$C_3$-Alkanoyl, $C_1$-$C_4$-Alkoxycarbonyl steht,

und

$R^2$ für H, Benzyl, $C_1$-$C_5$-Alkanoyl, gegebenenfalls ein- oder zweifach durch Halogen oder $C_1$-$C_4$-alkylsubstituiertes Benzoyl, $C_1$-$C_4$-Alkoxycarbonyl, Methansulfonyl, Benzolsulfonyl und Toluolsulfonyl steht.

**5.** Enantiomerenreine cis- Verbindungen der Formel (I) gemäß Anspruch 4
wobei

$R^1$ für H, Methyl, Benzyl, 1-Phenylethyl, Acetyl, Ethoxycarbonyl und t-Butoxycarbonyl steht

und

$R^2$ für H, Benzyl, Acetyl, Benzoyl, Ethoxycarbonyl, t-Butoxycarbonyl, Methansulfonyl und p-Toluolsulfonyl steht.

6. Racemische, cis-konfigurierte Verbindungen der Formel (I) gemäß Anspruch 4, wobei

$R^1$ für H, $C_1$-$C_3$-Alkyl, Phenyl, Benzyl, 1-Phenylethyl, $C_1$-$C_3$-Alkanoyl, $C_1$-$C_4$-Alkoxycarbonyl steht,

und

$R^2$ für H, Benzyl, $C_1$-$C_5$-Alkanoyl, gegebenenfalls ein- oder zweifach durch Halogen oder $C_1$-$C_4$-alkylsubstituiertes Benzoyl, $C_1$-$C_4$-Alkoxycarbonyl, Methansulfonyl, Benzolsulfonyl und Toluolsulfonyl steht,

mit Ausnahme von cis-5-Benzyl-8-tert.-butoxycarbonyl-2-oxa-5,8-diazabicyclo[4.3.0]nonan, cis-8-tert.-Butoxycarbonyl-2-oxa-5,8-diazabicyclo[4.3.0]nonan, cis-2-Oxa-5,8-diazabicyclo[4.3.0]-nonan.

7. Racemische, cis-konfigurierte Verbindungen der Formel (I) gemäß Anspruch 4, wobei

$R^1$ für H, Methyl, Benzyl, 1-Phenylethyl, Acetyl, Ethoxycarbonyl und t-Butoxycarbonyl steht

und

$R^2$ für H, Benzyl, Acetyl, Benzoyl, Ethoxycarbonyl, t-Butoxycarbonyl, Methansulfonyl und p-Toluolsulfonyl steht,

mit Ausnahme von cis-5-Benzyl-8-tert.-butoxycarbonyl-2-oxa-5,8-diazabicyclo[4.3.0]nonan, cis-8-tert.-Butoxycarbonyl-2-oxa-5,8-diazabicyclo[4.3.0]nonan, cis-2-Oxa-5,8-diazabicyclo[4.3.0]-nonan.

## Claims

1. Process for the preparation of enantiomerically pure cis compounds of the formula (I)

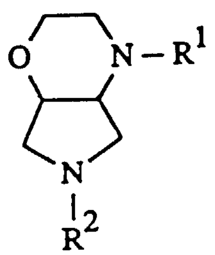

(I)

in which

$R^1$ represents H, $C_1$-$C_3$-alkyl, phenyl, benzyl, 1-phenylethyl, $C_1$-$C_3$-alkanoyl, $C_1$-$C_4$-alkoxycarbonyl

and

$R^2$ represents H, benzyl, $C_1$-$C_5$-alkanoyl, benzoyl which is optionally mono- or disubstituted by halogen or $C_1$-$C_4$-alkyl, $C_1$-$C_4$-alkoxycarbonyl, methanesulphonyl, benzenesulphonyl and toluenesulphonyl,

characterised in that 2,5-dihydropyrrole derivatives of the formula (II)

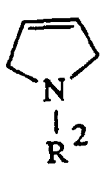

(II)

in which $R^2$ has the abovementioned meaning, with the exception of hydrogen, are reacted with compounds of

the formula (VIa)

$$Y\text{-}CH_2\text{-}CH_2\text{-}OR \qquad (VIa)$$

in which

Y denotes bromine, chlorine, hydroxyl or $-OSO-R^3$, in which $R^3$ denotes methyl, phenyl or methylphenyl

and

R denotes hydrogen

or

Y and R together denote a single bond

in the presence of bromine or N-bromosuccinimide to give racemic trans compounds of the formula (III)

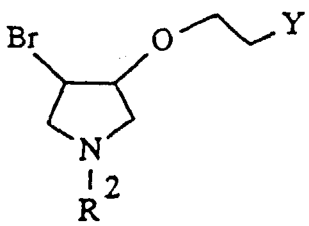

(III)

in which $R^2$ and Y have the abovementioned meaning, and it is possible if required to obtain, from compounds of the formula (III) in which Y denotes hydroxyl, the derivatives of the formula (III) in which Y has the other abovementioned meanings, according to generally known methods,
the compounds thus obtained of the formula (III) are either cyclised with amines of the formula (IV)

$$R^4\text{-}NH_2 \qquad (IV)$$

in which

$R^4$ represents hydrogen, $C_1$-$C_3$-alkyl, phenyl, benzyl or 1-phenylethyl,

to give racemic cis compounds of the formula (V)

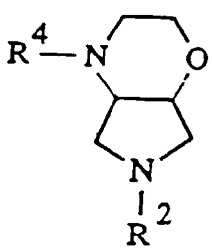

(V)

in which

$R^2$ and $R^4$ have the abovementioned meanings,

and radicals $R^2$ and/or $R^4$ optionally serving as protective groups are then removed using suitable methods such as reduction and/or hydro(geno)lysis and the secondary nitrogen atoms thus obtained are reacted again with compounds $R^1$-X and $R^2$-X, where X denotes a leaving group, and the racemates of the compounds of the formula (I) are resolved using methods known per se, and the enantiomeric compounds of the formulae (Ia-Ib) are isolated

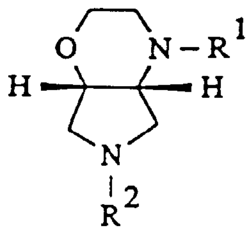

(Ia)

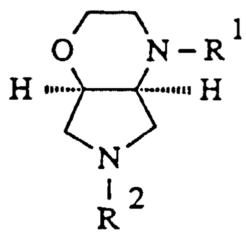

(Ib)

or racemic trans compounds of the formula (III) are reacted with enantiomerically pure R- or S-1-phenylethylamine to give mixtures of, in each case, two diastereomeric compounds of the formula (VII) in which $R^2$ has the above-mentioned meaning,

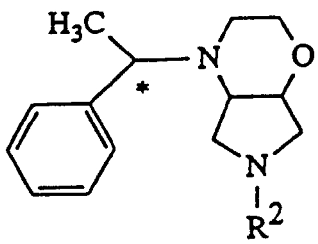

(VII)

the mixtures are separated into the enantiomerically pure compounds in a manner known per se, the phenylethyl substituent is cleaved off by hydrogenolysis if required, and a reaction is again carried out with compounds $R^2$-X, in which X denotes a leaving group, and the enantiomerically pure compounds of the formula (Ia-Ib) are isolated or by reacting S,S- or R,R-dihydroxypyrrolidine derivatives of the formula (VIIIa) or (VIIIb)

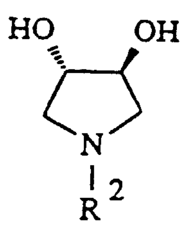

(VIIIa)

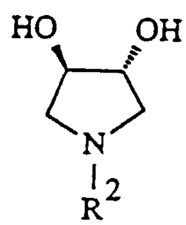

(VIIIb)

in which $R^2$ has the abovementioned meaning, but cannot be hydrogen, with allyl bromide or allyl chloride in the presence of a base to give compounds of the formula (IXa) or (IXb) respectively

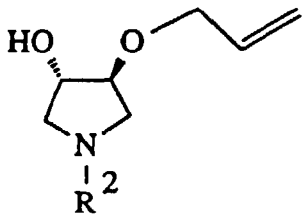

(IXa)

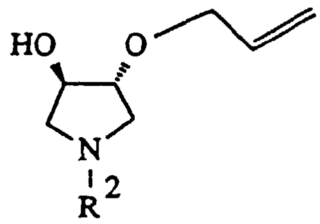

(IXb)

which are broken down, by ozonolysis and subsequent reduction, into compounds of the formula (Xa) and (Xb) respectively

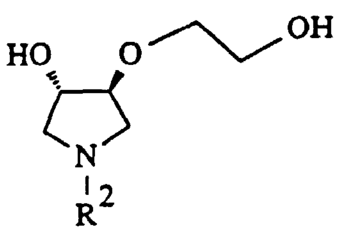

(Xa)

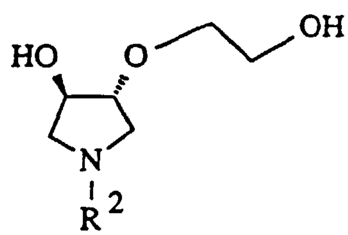

(Xb)

which, by acylation with sulphonyl chlorides $R^3$-$SO_2Cl$, are reacted to give sulphonic acid esters cf the formula (XIa) and (XIb) respectively, $R^3$ having the abovementioned meaning,

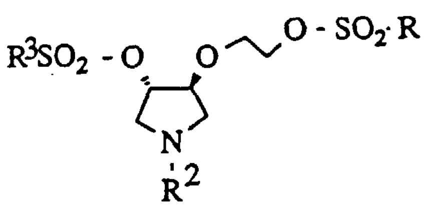

(XIa)

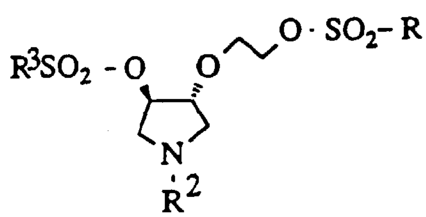

(XIb)

which, by reacting with amines of the formula (IV),

$$R^4\text{-}NH_2 \qquad (IV)$$

in which $R^4$ has the abovementioned meaning, are cyclised to give compounds of the formula (Va) and (Vb) respectively

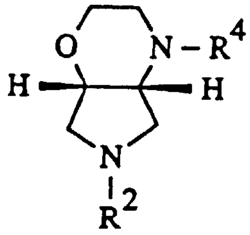

(Va)

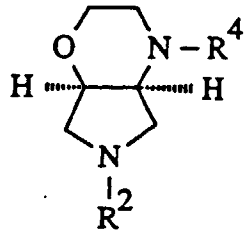

(Vb)

and radicals $R^2$ and/or $R^4$ optionally serving as protective groups then being removed using suitable methods such as reduction and/or hydro(geno)lysis and the secondary nitrogen atoms thus obtained being reacted again with compounds $R^1$-X and $R^2$-X, where X denotes a leaving group, and the racemates obtained of the compounds of the formula (I) being resolved using methods known per se, and the enantiomeric compounds of the formulae (Ia-Ib) being isolated.

**2.** Process according to Claim 1, characterised in that in formula (I)

$R^1$ represents H, methyl, benzyl, 1-phenylethyl, acetyl, ethoxycarbonyl and t-butoxycarbonyl

and

$R^2$ represents H, benzyl, acetyl, benzoyl, ethoxycarbonyl, t-butoxycarbonyl, methanesulphonyl and p-toluenesulphonyl.

**3.** Process according to Claims 1 and 2, characterised in that the reaction of the compounds of the formula (III) with amines of the formula (IV) is carried out in xylene or tetralin as the solvent.

**4.** Enantiomerically pure cis compounds of the formula (I)

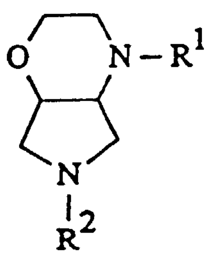

(I)

where

$R^1$ represents H, $C_1$-$C_3$-alkyl, phenyl, benzyl, 1-phenylethyl, $C_1$-$C_3$-alkanoyl, $C_1$-$C_4$-alkoxycarbonyl

and

$R^2$ represents H, benzyl, $C_1$-$C_5$-alkanoyl, benzoyl which is optionally mono- or disubstituted by halogen or $C_1$-$C_4$-alkyl, $C_1$-$C_4$-alkoxycarbonyl, methanesulphonyl, benzenesulphonyl and toluenesulphonyl.

**5.** Enantiomerically pure cis compounds of the formula (I) according to Claim 4, where

$R^1$ represents H, methyl, benzyl, 1-phenylethyl, acetyl, ethoxycarbonyl and t-butoxycarbonyl

and

$R^2$ represents H, benzyl, acetyl, benzoyl, ethoxycarbonyl, t-butoxycarbonyl, methanesulphonyl and p-toluenesulphonyl.

6. Racemic compounds of the formula (I) with the cis configuration according to Claim 4, where

$R^1$ represents H, $C_1$-$C_3$-alkyl, phenyl, benzyl, 1-phenylethyl, $C_1$-$C_3$-alkanoyl, $C_1$-$C_4$-alkoxycarbonyl

and

$R^2$ represents H, benzyl, $C_1$-$C_5$-alkanoyl, benzoyl which is optionally mono- or disubstituted by halogen or $C_1$-$C_4$-alkyl, $C_1$-$C_4$-alkoxycarbonyl, methanesulphonyl, benzenesulphonyl and toluenesulphonyl,

with the exception of cis-5-benzyl-8-tert-butoxycarbonyl-2-oxa-5,8-diazabicyclo[4.3.0]nonane, cis-8-tert-butoxycarbonyl-2-oxa-5,8-diazabicyclo[4.3.0]nonane and cis-2-oxa-5,8-diazabicyclo[4.3.0]nonane.

7. Racemic compounds of the formula (I) with the cis configuration according to Claim 4, where

$R^1$ represents H, methyl, benzyl, 1-phenylethyl, acetyl, ethoxycarbonyl and t-butoxycarbonyl

and

$R^2$ represents H, benzyl, acetyl, benzoyl, ethoxycarbonyl, t-butoxycarbonyl, methanesulphonyl and p-toluenesulphonyl.

with the exception of cis-5-benzyl-8-tert-butoxycarbonyl-2-oxa-5,8-diazabicyclo[4.3.0]nonane, cis-8-tert-butoxycarbonyl-2-oxa-5,8-diazabicyclo[4.3.0]nonane, cis-2-oxa-5,8-diazabicyclo[4.3.0]nonane.

**Revendications**

1. Procédé de production de composés cis-énantiomères purs de formule (I)

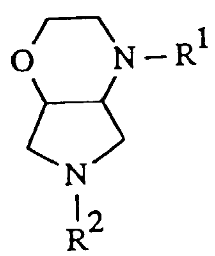

(I)

dans laquelle

$R^1$ représente H, un groupe alkyle en $C_1$ à $C_3$, phényle, benzyle, 1-phényléthyle, alcanoyle en $C_1$ à $C_3$, (alkoxy en $C_1$ à $C_4$)-carbonyle

et

$R^2$ représente H, des groupes benzyle, alcanoyle en $C_1$ à $C_5$, benzoyle éventuellement substitué une ou deux fois par un halogène ou un radical alkyle en $C_1$ à $C_4$, des groupes (alkoxy en $C_1$ à $C_4$)carbonyle, méthanesulfonyle, benzènesulfonyle et toluènesulfonyle,

caractérisé en ce qu'on fait réagir des dérivés de 2,5-dihydropyrrole de formule (II)

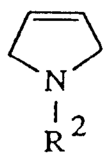

(II)

dans laquelle $R^2$ a la définition indiquée ci-dessus, excepté l'hydrogène, avec des composés de formule (VIa)

$$Y\text{-}CH_2\text{-}CH_2\text{-}OR \qquad \text{(VIa)}$$

dans laquelle

Y représente du brome, du chlore, un groupe hydroxy ou -OSO-$R^3$, où $R^3$ est un radical méthyle, phényle ou méthylphényle

et

R représente de l'hydrogène,

ou bien

Y et R représentent ensemble une liaison simple,

en présence de brome ou de N-bromosuccinimide pour former des composés trans-racémiques de formule (III)

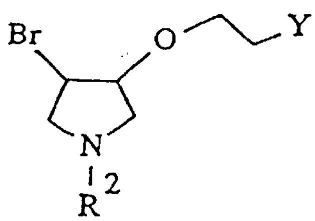

(III)

dans laquelle $R^2$ et Y ont la définition indiquée ci-dessus, les dérivés de formule (III) dans lesquels Y a les autres définitions indiquées ci-dessus pouvant éventuellement être obtenus par des procédés généralement connus à partir de composés de formule (III), dans lesquels Y est un groupe hydroxy,

on cyclise les composés ainsi obtenus de formule (III) avec des amines de formule (IV)

$$R^4\text{-}NH_2 \qquad \text{(IV)}$$

dans laquelle

$R^4$ représente de l'hydrogène, un groupe alkyle en $C_1$ à $C_3$, phényle, benzyle ou 1-phényléthyle,

pour former des composés cis-racémiques de formule (V)

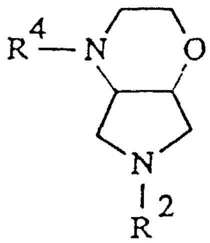

(V)

dans laquelle

$R^2$ et $R^4$ ont les définitions indiquées ci-dessus,

on élimine les restes $R^2$ et/ou $R^4$ servant éventuellement de groupes protecteurs par des procédés appréciés tels que réduction et/ou hydrolyse ou hydrogénolyse et on fait réagir de nouveau les atomes d'azote secondaires ainsi obtenus avec des composés $R^1$-X et $R^2$-X, dans lesquels X est un groupe partant, et on dédouble les racémates des composés de formule (I) par des procédés connus et on isole les composés énantiomères de formules (Ia-Ib)

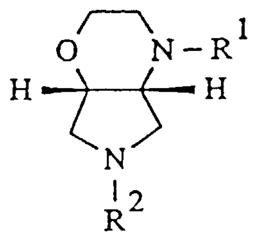

(Ia)

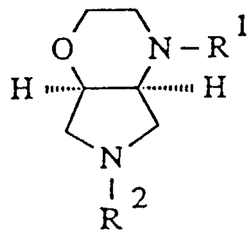

(Ib)

ou bien on fait réagir des composés trans-racémiques de formule (III) avec la R- ou S1-phényléthylamine énantiomère pure pour former des mélanges de chaque fois deux composés diastéréo-isomères de formule (VII)

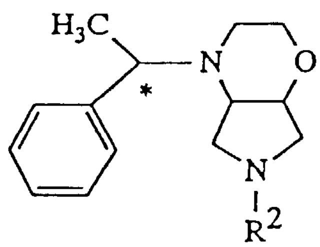

(VII)

dans laquelle $R^2$ a la définition indiquée ci-dessus,

on sépare les mélanges d'une manière connue en les composés énantiomères purs, on élimine éventuellement par hydrogénolyse le substituant phényléthyle et on conduit de nouveau une réaction avec des composés $R^2$-X dans lesquels X est un groupe partant et on isole les composés énantiomères purs de formule (Ia-Ib)

ou bien on fait réagir des dérivés de S,S- ou R,R-dihydroxypyrrolidine de formule (VIIIa) ou (VIIIb)

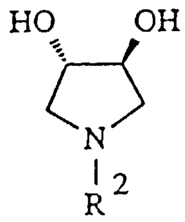

(VIIIa)

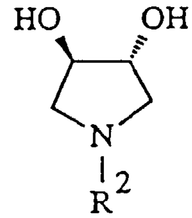

(VIIIb)

dans lesquels $R^2$ a la définition indiquée ci-dessus, mais ne peut pas représenter l'hydrogène, avec le bromure d'allyle ou le chlorure d'allyle en présence d'une base pour former les composés de formule respective (IXa) ou (IXb)

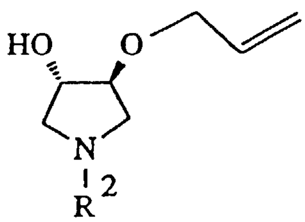

(IXa)

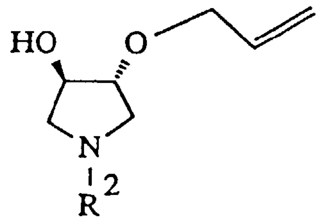

(IXb)

que l'on décompose par ozonolyse suivie d'une réduction en composés de formule respective (Xa) ou (Xb)

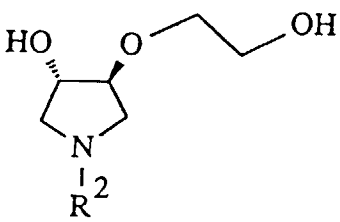

(Xa)

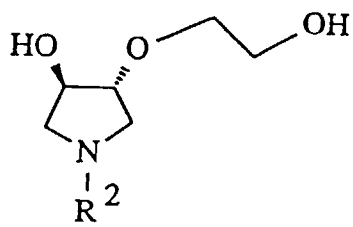

(Xb)

qu'on fait réagir par acylation avec des chlorures d'acide sulfonique $R^3$-$SO_2Cl$ pour former des esters d'acide sulfonique de formule respective (XIa) ou (XIb)

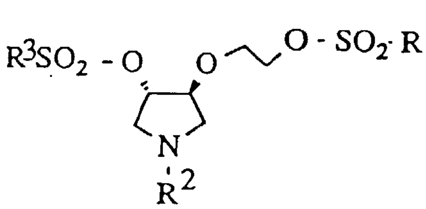

(XIa)

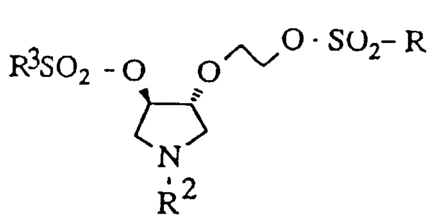

(XIb)

dans laquelle $R^3$ a la définition indiquée ci-dessus,

qu'on cyclise par réaction avec des amines de formule (IV)

$$R^4\text{-}NH_2 \qquad (IV)$$

dans laquelle $R^4$ a la définition indiquée ci-dessus, en composés de formule respective (Va) ou (Vb)

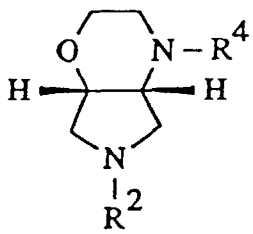

(Va)

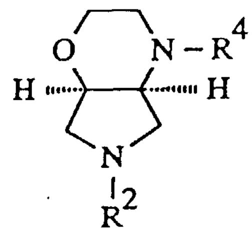

(Vb)

puis on élimine les restes $R^2$ et/ou $R^4$ servant éventuellement de groupe protecteur par des procédés appropriés tels que réduction et/ou hydrolyse ou hydrogénolyse et on fait réagir de nouveau les atomes d'azote secondaires ainsi obtenus avec des composés $R^1$-X et $R^2$-X, dans lesquels X est un groupe partant, et on dédouble par des procédés connus les racémates obtenus des composés de formule (I) et on isole les composés énantiomères de formules (Ia-Ib).

**2.** Procédé suivant la revendication 1, caractérisé en ce que dans la formule (I)

$R^1$ représente H, les groupes méthyle, benzyle, 1-phényléthyle, acétyle, éthoxycarbonyle et tertio-butoxycarbonyle

et

$R^2$ représente H, les groupes benzyle, acétyle, benzoyle, éthoxycarbonyle, tertio-butoxycarbonyle, méthanesulfonyle et p-toluènesulfonyle.

**3.** Procédé suivant les revendications 1 et 2, caractérisé en ce que la réaction des composés de formule (III) avec des amines de formule (IV) est conduite dans du xylène ou de la tétraline utilisé comme solvant.

**4.** Composés cis-énantiomères purs de formule (I)

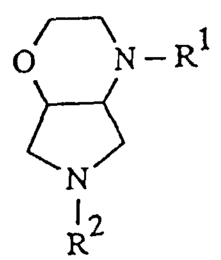

(I)

dans laquelle

$R^1$ représente H, un groupe alkyle en $C_1$ à $C_3$, phényle, benzyle, 1-phényléthyle, alcanoyle en $C_1$ à $C_3$, (alkoxy en $C_1$ à $C_4$)-carbonyle

et

$R^2$ représente H, des groupes benzyle, alcanoyle en $C_1$ à $C_5$, benzoyle éventuellement substitué une ou deux

fois par un halogène ou un radical alkyle en $C_1$ à $C_4$, des groupes (alkoxy en $C_1$ à $C_4$)carbonyle, méthanesulfonyle, benzènesulfonyle et toluènesulfonyle.

**5.** Composés cis-énantiomères purs de formule (I) suivant la revendication 4, dans lesquels

$R^1$ représente H, des groupes méthyle, benzyle, 1-phényléthyle, acétyle, éthoxycarbonyle et tertio-butoxycarbonyle

et

$R^2$ représente H, des groupes benzyle, acétyle, benzoyle, éthoxycarbonyle, tertio-butoxycarbonyle, méthanesulfonyle et p-toluènesulfonyle.

**6.** Composés racémiques à configuration cis de formule (I) suivant la revendication 4, dans lesquels

$R^1$ représente H, un groupe alkyle en $C_1$ à $C_3$, phényle, benzyle, 1-phényléthyle, alcanoyle en $C_1$ à $C_3$, (alkoxy en $C_1$ à $C_4$)-carbonyle

et

$R^2$ représente H, des groupes benzyle, alcanoyle en $C_1$ à $C_5$, benzoyle éventuellement substitué une ou deux fois par un halogène ou un radical alkyle en $C_1$ à $C_4$, des groupes (alkoxy en $C_1$ à $C_4$)carbonyle, méthanesulfonyle, benzènesulfonyle et toluènesulfonyle,

à l'exception du cis-5-benzyl-8-tertio-butoxycarbonyl-2-oxa-5,8-diazabicyclo[4.3.0]nonane, du cis-8-tertio-butoxycarbonyl-2-oxa-5,8-diazabicyclo[4.3.0]nonane et du cis-2-oxa-5,8-diazabicyclo[4.3.0]nonane.

**7.** Composés racémiques à configuration cis de formule (I) suivant la revendication 4, dans lesquels

$R^1$ représente H, des groupes méthyle, benzyle, 1-phényléthyle, acétyle, éthoxycarbonyle et tertio-butoxycarbonyle

et

$R^2$ représente H, des groupes benzyle, acétyle, benzoyle, éthoxycarbonyle, tertio-butoxycarbonyle, méthanesulfonyle et p-toluènesulfonyle,

à l'exception du cis-5-benzyl-8-tertio-butoxycarbonyl-2-oxa-5,8-diazabicyclo[4.3.0]nonane, du cis-8-tertio-butoxycarbonyl-2-oxa-5,8-diazabicyclo[4.3.0]nonane et du cis-2-oxa-5,8-diazabicyclo[4.3.0]nonane.